# EUROPEAN PATENT SPECIFICATION

(11) **EP 3 329 001 B1**
(45) Date of publication and mention of the grant of the patent: **22.09.2021**
(21) Application number: 16750569.2
(22) Date of filing: 28.07.2016
(51) Int. Cl.: C12N 15/10, C12N 15/90, C12N 9/22

(54) **GENOME EDITING SYSTEMS AND METHODS OF USE**
GENOMEDITINGSYSTEME UND VERFAHREN ZUR VERWENDUNG
SYSTÈMES D'ÉDITION DU GÉNOME ET MÉTHODES D'UTILISATION

(30) Priority: 28.07.2015 US 201562198049 P
(43) Date of publication of application: 06.06.2018
(73) Proprietor: Danisco US Inc., Palo Alto, California 94304 (US)
(72) Inventor: BAO, Kai, Palo Alto, California 94304 (US); MADRID, Susan Mampusti, Palo Alto, California 94304 (US); SCHMIDT, Brian F., Palo Alto, California 94304 (US)
(74) Representative: Mewburn Ellis LLP
(86) International application number: PCT/US2016/044489
(87) International publication number: WO 2017/019867

(56) References cited:
- WO-A1-2014/065596
- WO-A1-2014/102688
- WO-A1-2014/191518
- WO-A1-2015/054507
- WO-A1-2016/100272
- WO-A1-2016/100562
- WO-A1-2016/100568
- WO-A1-2016/100571
- WO-A1-2016/110453
- TORU MATSU-URA ET AL: "Efficient gene editing in Neurospora crassa with CRISPR technology", FUNGAL BIOL BIOTECHNOL, vol. 2, 15 July 2015 (2015-07-15), page 4, XP055268999, DOI: 10.1186/s40694-015-0015-1
- CHRISTINA S. NØDVIG ET AL: "A CRISPR-Cas9 System for Genetic Engineering of Filamentous Fungi", PLOS ONE, vol. 10, no. 7, 15 July 2015 (2015-07-15), page e0133085, XP55256394, DOI: 10.1371/journal.pone.0133085
- RUI LIU ET AL: "Efficient genome editing in filamentous fungus Trichoderma reesei using the CRISPR/Cas9 system", CELL DISCOVERY, vol. 1, 12 May 2015 (2015-05-12), page 15007, XP55263032, DOI: 10.1038/celldisc.2015.7
- TAKAYUKI ARAZOE ET AL: "Tailor-made CRISPR/Cas system for highly efficient targeted gene replacement in the rice blast fungus", BIOTECHNOLOGY AND BIOENGINEERING., vol. 112, no. 12, 14 July 2015 (2015-07-14), pages 2543-2549, XP55256237, US ISSN: 0006-3592, DOI: 10.1002/bit.25662
- TAKAYUKI ARAZOE ET AL: "Site-specific DNA double-strand break generated by I-SceI endonuclease enhances ectopic homologous recombination in Pyricularia oryzae", FEMS MICROBIOLOGY LETTERS, vol. 352, no. 2, 26 February 2014 (2014-02-26), pages 221-229, XP55256149, GB ISSN: 0378-1097, DOI: 10.1111/1574-6968.12396
- ZEHUA BAO ET AL: "Homology-Integrated CRISPR-Cas (HI-CRISPR) System for One-Step Multigene Disruption in Saccharomyces cerevisiae", ACS SYNTHETIC BIOLOGY, 10 September 2014 (2014-09-10), XP55175736, ISSN: 2161-5063, DOI: 10.1021/sb500255k
- FULLER K K ET AL: "Development of the CRISPR/Cas9 System for Targeted Gene Disruption in Aspergillus fumigatus", EUKARYOTIC CELL, AMERICAN SOCIETY FOR MICROBIOLOGY, US, vol. 14, no. 11, 1 November 2015 (2015-11-01), pages 1073-1080, XP002755315, ISSN: 1535-9778, DOI: 10.1128/EC.00107-15 [retrieved on 2015-08-28]
- CHI ZHANG ET AL: "Highly efficient CRISPR mutagenesis by microhomology-mediated end joining in Aspergillus fumigatus", FUNGAL GENETICS AND BIOLOGY, vol. 86, 1 January 2016 (2016-01-01), pages 47-57, XP55256363, US ISSN: 1087-1845, DOI: 10.1016/j.fgb.2015.12.007
- SCHUSTER MARIANA ET AL: "Genome editing in Ustilago maydis using the CRISPR-Cas system", FUNGAL GENETICS AND BIOLOGY, vol. 89, 11 September 2015 (2015-09-11), pages 3-9, XP029456293, ISSN: 1087-1845, DOI: 10.1016/J.FGB.2015.09.001
- J. E. DICARLO ET AL: "Genome engineering in Saccharomyces cerevisiae using CRISPR-Cas systems", NUCLEIC ACIDS RESEARCH, vol. 41, no. 7, 4 March 2013 (2013-03-04), pages 4336-4343, XP055086617, ISSN: 0305-1048, DOI: 10.1093/nar/gkt135
- IGOUCHEVA O ET AL: "Targeted gene correction by small single-stranded oligonucleotides in mammalian cells", GENE THERAPY, NATURE PUBLISHING GROUP, GB, vol. 8, no. 5, 1 March 2001 (2001-03-01), pages 391-399, XP001206900, ISSN: 0969-7128, DOI: 10.1038/SJ.GT.3301414
- PAWEL BIALK ET AL: "Regulation of Gene Editing Activity Directed by Single- Stranded Oligonucleotides and CRISPR/Cas9 Systems", PLOS ONE, vol. 10, no. 6, 8 June 2015 (2015-06-08), pages 1-19, XP055337921, DOI: 10.1371/journal.pone.0129308
- MASAFUMI INUI ET AL: "Rapid generation of mouse models with defined point mutations by the CRISPR/Cas9 system", SCIENTIFIC REPORTS, vol. 4, no. 1, 23 June 2014 (2014-06-23), XP055507692, DOI: 10.1038/srep05396
- WELD RICHARD J ET AL: "Approaches to functional genomics in filamentous fungi", CELL RESEARCH - XIBAO YANJIU, NATURE PUBLISHING GROUP, GB, CN, vol. 16, no. 1, 1 January 2006 (2006-01-01), pages 31-44, XP008092020, ISSN: 1001-0602, DOI: 10.1038/SJ.CR.7310006 [retrieved on 2006-01-16]

### SEQUENCE LISTING

<110> dANISCO
<120> GENOME EDITING SYSTEMS AND METHODS OF USE
<130> 40972-WO-PCT
<150> 62/198,049
   <151> 2015-07-28
<160> 33
<170> Patent In version 3.5
<210> 1
   <211> 70
   <212> DNA
   <213> Artificial Sequence
<220>
   <223> synthetic sequence
<400> 1
   gtcgtgctca agacgcacta cgacgtttaa accttaatta agcatggtct cgggctggga      60
   cttccacccg                                                             70
<210> 2
   <211> 70
   <212> DNA
   <213> Artificial Sequence
<220>
   <223> Synthetic Sequence
<400> 2
   cgggtggaag tcccagcccg agaccatgct taattaaggt ttaaacgtcg tagtgcgtct      60
   tgagcacgac                                                             70
<210> 3
   <211> 100
   <212> DNA
   <213> Artificial Sequence
<220>
   <223> synthetic sequence
<400> 3
   aagattggcc cgtcgattgt cgtgctcaag acgcactacg acgtttaaac cttaattaag      60
   catggtctcg ggctgggact tccacccgga gacgggcacg                           100
<210> 4
   <211> 100
   <212> DNA
   <213> Artificial Sequence
<220>
   <223> synthetic sequence
<400> 4
   cgtgcccgtc tccgggtgga agtcccagcc cgagaccatg cttaattaag gtttaaacgt      60
   cgtagtgcgt cttgagcacg acaatcgacg ggccaatctt                           100
<210> 5
   <211> 200
   <212> DNA
   <213> Artificial Sequence
<220>
   <223> synthetic sequence
<400> 5
   gcctgagcgc cgacgtgccg acagcgcgcg agctgctgta cctggccgac aagattggcc    60
   cgtcgattgt cgtgctcaag acgcactacg acgtttaaac cttaattaag catggtctcg   120
   ggctgggact tccacccgga gacgggcacg ggagcccagc tggcgtcgct ggcgcgcaag   180
   cacggcttcc tcatcttcga                                               200
<210> 6
   <211> 200
   <212> DNA
   <213> Artificial Sequence
<220>
   <223> synthetic sequence
<400> 6
   tcgaagatga ggaagccgtg cttgcgcgcc agcgacgcca gctgggctcc cgtgcccgtc    60
   tccgggtgga agtcccagcc cgagaccatg cttaattaag gtttaaacgt cgtagtgcgt   120
   cttgagcacg acaatcgacg ggccaatctt gtcggccagg tacagcagct cgcgcgctgt   180
   cggcacgtcg gcgctcaggc                                               200
<210> 7
   <211> 330
   <212> DNA
   <213> Artificial Sequence
<220>
   <223> synthetic sequence
<400> 7
   gcggccagga gcgagacggc gacgcacccg ctgacggctt acctgttcaa gctcatggac    60
   ctcaaggcgt ccaacctgtg cctgagcgcc gacgtgccga cagcgcgcga gctgctgtac   120
   ctggccgaca agattggccc gtcgattgtc gtcgtttaaa ccttaattaa gtctcgggct   180
   gggacttcca cccggagacg ggcacgggag cccagctggc gtcgctggcg cgcaagcacg   240
   gcttcctcat cttcgaggac cgcaagtttg gcgacattgg ccacaccgtc gagctgcagt   300
   acacgggcgg gtcggcgcgc atcatcgact                                    330
<210> 8
   <211> 730
   <212> DNA
   <213> Artificial Sequence
<220>
   <223> synthetic sequence
<400> 8
   ctactcctct tcctcccgca cggctgtcgc gcttccaacg tcattcgcac agcagaattg      60
   tgccatctct ctcttttttt tccccccctc taaaccgcca caacggcacc ctaagggtta    120
   aactatccaa ccagccgcag cctcagcctc tctcagcctc atcagccatg gcaccacacc     180
   cgacgctcaa ggccaccttc gcggccagga gcgagacggc gacgcacccg ctgacggctt     240
   acctgttcaa gctcatggac ctcaaggcgt ccaacctgtg cctgagcgcc gacgtgccga     300
   cagcgcgcga gctgctgtac ctggccgaca agattggccc gtcgattgtc gtcgtttaaa     360
   ccttaattaa gtctcgggct gggacttcca cccggagacg ggcacgggag cccagctggc     420
   gtcgctggcg cgcaagcacg gcttcctcat cttcgaggac cgcaagtttg gcgacattgg     480
   ccacaccgtc gagctgcagt acacgggcgg gtcggcgcgc atcatcgact gggcgcacat     540
   tgtcaacgtc aacatggtgc ccggcaaggc gtcggtggcc tcgctggccc agggcgccaa     600
   gcgctggctc gagcgctacc cctgcgaggt caagacgtcc gtcaccgtcg gcacgcccac     660
   catggactcg tttgacgacg acgccgactc cagggacgcc gagcccgccg gcgccgtcaa     720
   cggcatgggc                                                            730
<210> 9
   <211> 1100
   <212> DNA
   <213> Artificial Sequence
<220>
   <223> synthetic sequence
<400> 9
   ctcaccccca aagtcgcaat atcgggtatc gccgccggca ttgaatcgcc ttctccgcta      60
   gcatcgacta ctgctgctct gctctcgttg ccagcgctgc tccctagaat tttgaccagg     120
   ggacgagccc gacattaaag caactccctc gcctcgagac gactcggatc gcacgaaatt     180
   ctcccaatcg ccgacagttc ctactcctct tcctcccgca cggctgtcgc gcttccaacg     240
   tcattcgcac agcagaattg tgccatctct ctcttttttt tccccccctc taaaccgcca    300
   caacggcacc ctaagggtta aactatccaa ccagccgcag cctcagcctc tctcagcctc     360
   atcagccatg gcaccacacc cgacgctcaa ggccaccttc gcggccagga gcgagacggc     420
   gacgcacccg ctgacggctt acctgttcaa gctcatggac ctcaaggcgt ccaacctgtg     480
   cctgagcgcc gacgtgccga cagcgcgcga gctgctgtac ctggccgaca agattggccc 540
   gtcgattgtc gtcgtttaaa ccttaattaa gtctcgggct gggacttcca cccggagacg 600
   ggcacgggag cccagctggc gtcgctggcg cgcaagcacg gcttcctcat cttcgaggac 660
   cgcaagtttg gcgacattgg ccacaccgtc gagctgcagt acacgggcgg gtcggcgcgc 720
   atcatcgact gggcgcacat tgtcaacgtc aacatggtgc ccggcaaggc gtcggtggcc 780
   tcgctggccc agggcgccaa gcgctggctc gagcgctacc cctgcgaggt caagacgtcc 840
   gtcaccgtcg gcacgcccac catggactcg tttgacgacg acgccgactc cagggacgcc 900
   gagcccgccg gcgccgtcaa cggcatgggc tccattggcg tcctggacaa gcccatctac 960
   tcgaaccggt ccggcgacgg ccgcaagggc agcatcgtct ccatcaccac cgtcacccag 1020
   cagtacgagt ccgtctcctc gccccggtta acaaaggcca tcgccgaggg cgacgagtcg 1080
   ctcttcccgg gcatcgagga 1100
<210> 10
   <211> 23
   <212> DNA
   <213> Artificial Sequence
<220>
   <223> synthetic sequence
<400> 10
   gctcaagacg cactacgaca tgg 23
<210> 11
   <211> 131
   <212> DNA
   <213> Artificial Sequence
<220>
   <223> synthetic
<400> 11
   cgcgaaatta atacgactca ctatagggct caagacgcac tacgacagtt ttagagctag 60
   aaatagcaag ttaaaataag gctagtccgt tatcaacttg aaaaagtggc accgagtcgg 120
   tgctttttag c 131
<210> 12
   <211> 26
   <212> DNA
   <213> Artificial Sequence
<220>
   <223> primer
<400> 12
   ccatcttggc tgacgaaaaa ggtctg 26
<210> 13
   <211> 29
   <212> DNA
   <213> Artificial Sequence
<220>
   <223> primer
<400> 13
   catgcaaaga tacacatcaa tcgcagctg 29
<210> 14
   <211> 23
   <212> DNA
   <213> Artificial Sequence
<220>
   <223> primer
<400> 14
   catcgactac tgctgctctg ctc 23
<210> 15
   <211> 23
   <212> DNA
   <213> Artificial Sequence
<220>
   <223> primer
<400> 15
   atcgcagctg gggtacaatc atc 23
<210> 16
   <211> 18
   <212> DNA
   <213> Artificial Sequence
<220>
   <223> primer
<400> 16
   cggcgatggc ctttgtta 18
<210> 17
   <211> 100
   <212> DNA
   <213> Artificial Sequence
<220>
   <223> synthetic sequence
<400> 17
   atcagccgtg cgtgtgacca gtgtaaccaa ctccgaacga aatgcgttta aaccttaatt 60
   aagcgacggg cagcatccgt gcgctcattg cattggtagg 100
<210> 18
   <211> 100
   <212> DNA
   <213> Artificial Sequence
<220>
   <223> synthetic sequence
<400> 18
   cctaccaatg caatgagcgc acggatgctg cccgtcgctt aattaaggtt taaacgcatt 60
   tcgttcggag ttggttacac tggtcacacg cacggctgat 100
<210> 19
   <211> 200
   <212> DNA
   <213> Artificial Sequence
<220>
   <223> synthetic sequence
<400> 19
   ctcgctctgc cgtccgcaaa acctcgtctt cagctccggt tcgccgccga atcagccgtg 60
   cgtgtgacca gtgtaaccaa ctccgaacga aatgcgttta aaccttaatt aagcgacggg 120
   cagcatccgt gcgctcattg cattggtagg cttccgctct ttctccgatg ccggcgatga 180
   ggcggacgct tgactgacct 200
<210> 20
   <211> 200
   <212> DNA
   <213> Artificial Sequence
<220>
   <223> synthetic sequence
<400> 20
   aggtcagtca agcgtccgcc tcatcgccgg catcggagaa agagcggaag cctaccaatg 60
   caatgagcgc acggatgctg cccgtcgctt aattaaggtt taaacgcatt tcgttcggag 120
   ttggttacac tggtcacacg cacggctgat tcggcggcga accggagctg aagacgaggt 180
   tttgcggacg gcagagcgag 200
<210> 21
   <211> 19
   <212> DNA
   <213> Artificial Sequence
<220>
   <223> synthetic sequence
<400> 21
   cgtttaaacc ttaattaag 19
<210> 22
   <211> 20
   <212> DNA
   <213> Artificial Sequence
<220>
   <223> synthetic sequence
<400> 22
   caactccgaa cgaaatgcga 20
<210> 23
   <211> 132
   <212> DNA
   <213> Artificial Sequence
<220>
   <223> synthetic sequence
<400> 23
   cgcgaaatta atacgactca ctataggcaa ctccgaacga aatgcgagtt ttagagctag 60
   aaatagcaag ttaaaataag gctagtccgt tatcaacttg aaaaagtggc accgagtcgg 120
   tgcttttttt aa 132
<210> 24
   <211> 29
   <212> DNA
   <213> Artificial Sequence
<220>
   <223> primer
<400> 24
   ggcttccccg cctttttccc ctgcaattc 29
<210> 25
   <211> 36
   <212> DNA
   <213> Artificial Sequence
<220>
   <223> primer
<400> 25
   atgccacccc tcttgacaat gaagccagca taagcc 36
<210> 26
   <211> 4197
   <212> DNA
   <213> Streptococcus pyogenes
<400> 26
   catatggccc caaaaaagaa acgcaaggtt atggataaaa aatacagcat tggtctggat       60
   atcggaacca acagcgttgg gtgggcagta ataacagatg aatacaaagt gccgtcaaaa      120
   aaatttaagg ttctggggaa tacagatcgc cacagcataa aaaagaatct gattggggca      180
   ttgctgtttg attcgggtga gacagctgag gccacgcgtc tgaaacgtac agcaagaaga 240
   cgttacacac gtcgtaaaaa tcgtatttgc tacttacagg aaattttttc taacgaaatg 300
   gccaaggtag atgatagttt cttccatcgt ctcgaagaat cttttctggt tgaggaagat 360
   aaaaaacacg aacgtcaccc tatctttggc aatatcgtgg atgaagtggc ctatcatgaa 420
   aaatacccta cgatttatca tcttcgcaag aagttggttg atagtacgga caaagcggat 480
   ctgcgtttaa tctatcttgc gttagcgcac atgatcaaat ttcgtggtca tttcttaatt 540
   gaaggtgatc tgaatcctga taactctgat gtggacaaat tgtttataca attagtgcaa 600
   acctataatc agctgttcga ggaaaacccc attaatgcct ctggagttga tgccaaagcg 660
   attttaagcg cgagactttc taagtcccgg cgtctggaga atctgatcgc ccagttacca 720
   ggggaaaaga aaaatggtct gtttggtaat ctgattgccc tcagtctggg gcttaccccg 780
   aacttcaaat ccaattttga cctggctgag gacgcaaagc tgcagctgag caaagatact 840
   tatgatgatg acctcgacaa tctgctcgcc cagattggtg accaatatgc ggatctgttt 900
   ctggcagcga agaatctttc ggatgctatc ttgctgtcgg atattctgcg tgttaatacc 960
   gaaatcacca aagcgcctct gtctgcaagt atgatcaaga gatacgacga gcaccaccag 1020
   gacctgactc ttcttaaggc actggtacgc caacagcttc cggagaaata caaagaaata 1080
   ttcttcgacc agtccaagaa tggttacgcg ggctacatcg atggtggtgc atcacaggaa 1140
   gagttctata aatttattaa accaatcctt gagaaaatgg atggcacgga agagttactt 1200
   gttaaactta accgcgaaga cttgcttaga aagcaacgta cattcgacaa cggctccatc 1260
   ccacaccaga ttcatttagg tgaacttcac gccatcttgc gcagacaaga agatttctat 1320
   cccttcttaa aagacaatcg ggagaaaatc gagaagatcc tgacgttccg cattccctat 1380
   tatgtcggtc ccctggcacg tggtaattct cggtttgcct ggatgacgcg caaaagtgag 1440
   gaaaccatca ccccttggaa ctttgaagaa gtcgtggata aaggtgctag cgcgcagtct 1500
   tttatagaaa gaatgacgaa cttcgataaa aacttgccca acgaaaaagt cctgcccaag 1560
   cactctcttt tatatgagta ctttactgtg tacaacgaac tgactaaagt gaaatacgtt 1620
   acggaaggta tgcgcaaacc tgcctttctt agtggcgagc agaaaaaagc aattgtcgat 1680
   cttctcttta aaacgaatcg caaggtaact gtaaaacagc tgaaggaaga ttatttcaaa 1740
   aagatcgaat gctttgattc tgtcgagatc tcgggtgtcg aagatcgttt caacgcttcc 1800
   ttagggacct atcatgattt gctgaagata ataaaagaca aagactttct cgacaatgaa 1860
   gaaaatgaag atattctgga ggatattgtt ttgaccttga ccttattcga agatagagag 1920
   atgatcgagg agcgcttaaa aacctatgcc cacctgtttg atgacaaagt catgaagcaa 1980
   ttaaagcgcc gcagatatac ggggtggggc cgcttgagcc gcaagttgat taacggtatt 2040
   agagacaagc agagcggaaa aactatcctg gatttcctca aatctgacgg atttgcgaac 2100
   cgcaatttta tgcagcttat acatgatgat tcgcttacat tcaaagagga tattcagaag 2160
   gctcaggtgt ctgggcaagg tgattcactc cacgaacata tagcaaattt ggccggctct 2220
   cctgcgatta agaaggggat cctgcaaaca gttaaagttg tggatgaact tgtaaaagta 2280
   atgggccgcc acaagccgga gaatatcgtg atagaaatgg cgcgcgagaa tcaaacgaca 2340
   caaaaaggtc aaaagaactc aagagagaga atgaagcgca ttgaggaggg gataaaggaa 2400
   cttggatctc aaattctgaa agaacatcca gttgaaaaca ctcagctgca aaatgaaaaa 2460
   ttgtacctgt actacctgca gaatggaaga gacatgtacg tggatcagga attggatatc 2520
   aatagactct cggactatga cgtagatcac attgtccctc agagcttcct caaggatgat 2580
   tctatagata ataaagtact tacgagatcg gacaaaaatc gcggtaaatc ggataacgtc 2640
   ccatcggagg aagtcgttaa aaagatgaaa aactattggc gtcaactgct gaacgccaag 2700
   ctgatcacac agcgtaagtt tgataatctg actaaagccg aacgcggtgg tcttagtgaa 2760
   ctcgataaag caggatttat aaaacggcag ttagtagaaa cgcgccaaat tacgaaacac 2820
   gtggctcaga tcctcgattc tagaatgaat acaaagtacg atgaaaacga taaactgatc 2880
   cgtgaagtaa aagtcattac cttaaaatct aaacttgtgt ccgatttccg caaagatttt 2940
   cagttttaca aggtccggga aatcaataac tatcaccatg cacatgatgc atatttaaat 3000
   gcggttgtag gcacggccct tattaagaaa taccctaaac tcgaaagtga gtttgtttat 3060
   ggggattata aagtgtatga cgttcgcaaa atgatcgcga aatcagaaca ggaaatcggt 3120
   aaggctaccg ctaaatactt tttttattcc aacattatga atttttttaa gaccgaaata 3180
   actctcgcga atggtgaaat ccgtaaacgg cctcttatag aaaccaatgg tgaaacggga 3240
   gaaatcgttt gggataaagg tcgtgacttt gccaccgttc gtaaagtcct ctcaatgccg 3300
   caagttaaca ttgtcaagaa gacggaagtt caaacagggg gattctccaa agaatctatc 3360
   ctgccgaagc gtaacagtga taaacttatt gccagaaaaa aagattggga tccaaaaaaa 3420
   tacggaggct ttgattcccc taccgtcgcg tatagtgtgc tggtggttgc taaagtcgag 3480
   aaagggaaaa gcaagaaatt gaaatcagtt aaagaactgc tgggtattac aattatggaa 3540
   agatcgtcct ttgagaaaaa tccgatcgac tttttagagg ccaaggggta taaggaagtg 3600
   aaaaaagatc tcatcatcaa attaccgaag tatagtcttt ttgagctgga aaacggcaga 3660
   aaaagaatgc tggcctccgc gggcgagtta cagaagggaa atgagctggc gctgccttcc 3720
   aaatatgtta attttctgta ccttgccagt cattatgaga aactgaaggg cagccccgaa 3780
   gataacgaac agaaacaatt attcgtggaa cagcataagc actatttaga tgaaattata 3840
   gagcaaatta gtgaattttc taagcgcgtt atcctcgcgg atgctaattt agacaaagta 3900
   ctgtcagctt ataataaaca tcgggataag ccgattagag aacaggccga aaatatcatt 3960
   catttgttta ccttaaccaa ccttggagca ccagctgcct tcaaatattt cgataccaca 4020
   attgatcgta aacggtatac aagtacaaaa gaagtcttgg acgcaaccct cattcatcaa 4080
   tctattactg gattatatga gacacgcatt gatctttcac agctgggcgg agacaagaag 4140
   aaaaaactga aactgcacca tcatcaccat catcatcacc atcattgata actcgag 4197
<210> 27
   <211> 270
   <212> DNA
   <213> Bacillus subtilis
<400> 27
   atgaaatcta aatggatgtc aggtttgttg ctcgttgcgg tcgggttcag ctttactcag 60
   gtgatggttc tactttagat ttacctacag tccaaacaac gagcaacgcc agcccaagtc 120
   gaaatgagtc cactaccaag atgcaggtga aacagcaaac acagaaggga aaacatttca 180
   tattgcggca cgcaatcaaa catgatacgt ccactttgtc gtttgtgtct tcccttttgt 240
   aaagtataac gccgtgcgtt agtttgtact 270
<210> 28
   <211> 23
   <212> DNA
   <213> Bacillus subtilis
<400> 28
   actcaggtga tggttcatgc agg 23
<210> 29
   <211> 27
   <212> DNA
   <213> Artificial Sequence
<220>
   <223> primer
<400> 29
   atgaaatcta aatggatgtc aggtttg 27
<210> 30
   <211> 27
   <212> DNA
   <213> Artificial Sequence
<220>
   <223> primer
<400> 30
   tcatgtttga ttgcgtgccg caatatg 27
<210> 31
   <211> 4111
   <212> DNA
   <213> Streptococcus pyogenes
<400> 31
   atggacaaga agtactccat cggcctggac atcggcacca actccgtcgg ctgggccgtc 60
   atcacggacg agtacaaggt cccgtcgaag aagttcaagg tgctgggcaa cacggaccgc 120
   cacagcatca agaagaacct catcggcgcc ctcctgttcg acagcggcga gacggccgag 180
   gcgacccgcc tgaagcgcac ggcgcggcgg cggtacacgc gccgcaagaa ccgcatctgc 240
   tacctccagg agatcttctc caacgagatg gcgaaggtcg acgactcgtt cttccaccgg 300
   ctggaggagt ccttcctggt cgaggaggac aagaagcacg agcggcaccc catcttcggc 360
   aacatcgtgg acgaggtggc ctaccacgag aagtacccga ccatctacca cctccggaag 420
   aagctggtgg actcgaccga caaggccgac ctgcgcctga tctacctggc cctcgcccac 480
   atgatcaagt tccgcggcca cttcctcatc gagggcgacc tgaacccgga caactcggac 540
   gtcgacaagc tgttcatcca gctcgtgcag acctacaacc agctcttcga ggagaacccg 600
   atcaacgcct ccggcgtgga cgcgaaggcc atcctgtcgg cccgcctctc gaagtcccgg 660
   cggctcgaga acctgatcgc ccagctgccg ggcgagaaga agaacggcct cttcggcaac 720
   ctgatcgcgc tctccctggg cctgacgccg aacttcaagt ccaacttcga cctcgccgag 780
   gacgccaagc tccagctgtc caaggacacg tacgacgacg acctggacaa cctcctggcg 840
   cagatcggcg accagtacgc ggacctgttc ctcgcggcga agaacctctc cgacgcgatc 900
   ctgctcagcg acatcctccg cgtcaacacc gagatcacca aggcgcccct gtccgcgagc 960
   atgatcaagc gctacgacga gcaccaccag gacctcaccc tgctgaaggc cctggtccgc 1020
   cagcagctcc cggagaagta caaggagatc ttcttcgacc agtccaagaa cggctacgcg 1080
   ggctacatcg acggcggcgc gtcccaggag gagttctaca agttcatcaa gcccatcctg 1140
   gagaagatgg acggcaccga ggagctgctg gtcaagctga accgggagga cctcctgcgc 1200
   aagcagcgca cgttcgacaa cggctccatc ccccaccaga tccacctggg cgagctgcac 1260
   gccatcctcc ggcgccagga ggacttctac ccgttcctca aggacaaccg cgagaagatc 1320
   gagaagatcc tcacgttccg gatcccgtac tacgtcggcc ccctcgcccg cggcaactcc 1380
   cgcttcgcgt ggatgacgcg caagtcggag gagacgatca ccccgtggaa cttcgaggag 1440
   gtggtggaca agggcgcctc cgcgcagtcg ttcatcgagc ggatgaccaa cttcgacaag 1500
   aacctgccga acgagaaggt gctgcccaag cacagcctcc tgtacgagta cttcacggtc 1560
   tacaacgagc tcaccaaggt gaagtacgtg acggagggca tgcggaagcc ggcgttcctg 1620
   tccggcgagc agaagaaggc catcgtggac ctgctgttca agacgaaccg gaaggtcacc 1680
   gtcaagcagc tgaaggagga ctacttcaag aagatcgagt gcttcgactc ggtcgagatc 1740
   agcggcgtgg aggaccgctt caacgcgtcg ctgggcacgt accacgacct cctgaagatc 1800
   atcaaggaca aggacttcct cgacaacgag gagaacgagg acatcctgga ggacatcgtg 1860
   ctgaccctga cgctcttcga ggaccgggag atgatcgagg agcgcctcaa gacctacgcg      1920
   cacctgttcg acgacaaggt catgaagcag ctcaagcggc ggcggtacac cggctggggc      1980
   cggctctccc gcaagctcat caacggcatc cgggacaagc agtcgggcaa gaccatcctc      2040
   gacttcctga agtccgacgg cttcgccaac cgcaacttca tgcagctgat ccacgacgac      2100
   agcctgacgt tcaaggagga catccagaag gcgcaggtgt cgggccaggg cgactccctg      2160
   cacgagcaca tcgcgaacct ggccggcagc ccggccatca agaagggcat cctgcagacg      2220
   gtcaaggtgg tcgacgagct ggtgaaggtg atgggccgcc acaagcccga gaacatcgtc      2280
   atcgagatgg cccgggagaa ccagaccacg cagaagggcc agaagaacag ccgcgagcgc      2340
   atgaagcgga tcgaggaggg catcaaggag ctcggctcgc agatcctcaa ggagcacccc      2400
   gtcgagaaca cccagctcca gaacgagaag ctctacctct actacctgca gaacggccgg      2460
   gacatgtacg tggaccagga gctcgacatc aaccggctgt cggactacga cgtcgaccac      2520
   atcgtcccgc agtcgttcct gaaggacgac agcatcgaca acaaggtcct cacccgctcg      2580
   gacaagaacc ggggcaagtc cgacaacgtc ccgtcggagg aggtcgtgaa gaagatgaag      2640
   aactactggc ggcagctcct caacgccaag ctcatcacgc agcgcaagtt cgacaacctg      2700
   acgaaggcgg agcggggcgg cctcagcgag ctcgacaagg cgggcttcat caagcggcag      2760
   ctggtcgaga cgcggcagat cacgaagcac gtggcccaga tcctggactc gcgcatgaac      2820
   accaagtacg acgagaacga caagctgatc cgcgaggtca aggtcatcac cctcaagtcc      2880
   aagctggtga gcgacttccg caaggacttc cagttctaca aggtgcggga gatcaacaac      2940
   taccaccacg cccacgacgc gtacctgaac gcggtggtgg gcacggcgct gatcaagaag      3000
   tacccgaagc tcgagagcga gttcgtctac ggcgactaca aggtgtacga cgtgcggaag      3060
   atgatcgcga agagcgagca ggagatcggc aaggccacgg cgaagtactt cttctacagc      3120
   aacatcatga acttcttcaa gaccgagatc accctggcga acggcgagat ccgcaagcgc      3180
   ccgctcatcg agaccaacgg cgagacgggc gagatcgtgt gggacaaggg ccgcgacttc      3240
   gcgaccgtcc ggaaggtgct gtccatgccc caggtcaaca tcgtcaagaa gacggaggtc      3300
   cagaccggcg gcttcagcaa ggagtccatc ctgcccaagc ggaacagcga caagctgatc      3360
   gcccggaaga aggactggga ccccaagaag tacggcggct tcgactcccc caccgtcgcc      3420
   tactcggtcc tcgtggtcgc caaggtggag aagggcaaga gcaagaagct gaagtccgtg      3480
   aaggagctgc tgggcatcac catcatggag cggtcgtcgt tcgagaagaa ccccatcgac      3540
   ttcctggagg ccaagggcta caaggaggtg aagaaggacc tcatcatcaa gctccccaag      3600
   tactccctgt tcgagctgga gaacggccgg aagcgcatgc tcgccagcgc gggcgagctg      3660
   cagaagggca acgagctggc gctgccgagc aagtacgtca acttcctgta cctcgcctcg      3720
   cactacgaga agctgaaggg ctcgccggag gacaacgagc agaagcagct gttcgtggag     3780
   cagcacaagc actacctcga cgagatcatc gagcagatct ccgagttctc caagcgcgtc     3840
   atcctggccg acgcgaacct ggacaaggtc ctgtccgcgt acaacaagca ccgcgacaag     3900
   ccgatccggg agcaggccga gaacatcatc cacctgttca cgctgaccaa cctcggcgcc     3960
   cccgcggcct tcaagtactt cgacaccacg atcgaccgga agcggtacac gagcacgaag     4020
   gaggtgctcg acgccaccct gatccaccag agcatcacgg gcctgtacga gacccgcatc     4080
   gacctctccc agctgggcgg cgactgatag a                                   4111
<210> 32
   <211> 20
   <212> DNA
   <213> Artificial Sequence
<220>
   <223> synthetic sequence
<400> 32
   ttcatcgagt cctaccgtga 20
<210> 33
   <211> 400
   <212> DNA
   <213> Artificial Sequence
<220>
   <223> synthetic sequence
<400> 33
   cgacctgacc ccggacacgc tgccctactg ggagctgcgg gccacgtcct cgctggtcac       60
   cggaatcgag aaggcgttca tcgagtccta ccgcgtttaa gctggactgg ggcctgtgcg      120
   acgggatgac cctcgacgcc cggtgcagga gcgaccagtg gccttagctc ttccgcaagt      180
   agctcaggat ggcgcaaatt accttaatta agtgacggct ccttccagta cgtcctgatc      240
   gcggccgacc gcgtgtgagc gacgcggacc ccggctgaac gactggtccc agcacgatgc      300
   tggaattaat tcactgccga ggaaggtcat gcaggactag cgccggctgg cgcacactcg      360
   ctgcgcctgg ggccgacttg ctgaccaggg tcgtgctacg                            400

## Description

### FIELD OF THE INVENTION

The present disclosure is generally related to the fields of molecular biology, genetics, biochemistry, genome editing and filamentous fungi.

### BACKGROUND

It is known that inducing cleavage at a specific target site in genomic DNA can be used to introduce modifications at or near the target site. For example, homologous recombination for gene targeting has been shown to be enhanced when the targeted DNA site contains a double-strand break (*see, e.g.,* Rudin *et al., 1989;* Smith *et al*.).

Given the site-specific nature of Cas systems, genome modification/engineering techniques based on these systems have been described, including in mammalian cells (*see, e.g.,* Hsu *et al.,* 2014). Cas-based genome engineering, when functioning as intended, confers the ability to target virtually any specific location within a complex genome, by designing a recombinant crRNA (or equivalently functional polynucleotide) in which the DNA-targeting region (*i.e.,* the variable targeting domain) of the crRNA is homologous to a desired target site in the genome, and combining the crRNA with a Cas endonuclease (through any convenient and conventional means) into a functional complex in a host cell.

Although Cas-based genome engineering techniques have been applied to a number of different host cell types, even in filamentous fungal cells (*see, e.g.,* Liu *et al.,* 2015), these techniques have known limitations. For example, the efficiency of gene editing is strain-dependent, and generally multiple steps of molecular manipulation are required for donor DNA construction and the like.

Therefore, there remains a need for developing more effective, efficient or otherwise more robust or flexible Cas-based genome editing methods and compositions thereof for modifying/altering a genomic target site in a microbial cell.

WO 2015/054507 discloses methods for the production of nutritive polypeptides in fungi, in particular Aspergillus, where the expression of said polypeptide is increased by co-expressing in the fungal host (i) a variant nucleic acid encoding a secreted protease and (ii) a nucleic acid encoding a Cas9 endonuclease plus a nucleic acid encoding a guide RNA.

Matsu-ura *et al.* (2015) reports on gene replacement in the model filamentous fungus *Neurospora crassa* with the CRISPR/Cas system.

Nødvig *et al.* (2015) describes a CRISPR/Cas9 based system adapted for use in filamentous fungi and performs RNA-guided mutagenesis in six species.

Arazoe et al. (2015) discloses the establishment of the CRISPR/Cas system in the model filamentous fungus *Pyricularia oryzae* and demonstrates the system could recognise the desired sequence and edit endogenous genes through homologous recombination-mediated targeted gene replacement.

Arazoe et al. (2014) reports on the establishment of a detection/selection system of double-strand break mediated ectopic homologous recombination in *Pyricularia oryzae.*

WO 2014/191518 discloses a method for genome engineering based on the type II CRISPR system that involves the use of nickase architectures of Cas9 and single or multiple crRNA(s) harbouring two different targets.

DiCarlo *et al.* (2013) reports on the use of CRISPR/Cas system in *Saccharomyces cerevisiae* for genome engineering.

### SUMMARY

The invention provides a method for genome editing in a filamentous fungal cell, comprising introducing into the filamentous fungal cell a Cas endonuclease, a guide polynucleotide, and a donor polynucleotide, wherein the donor polynucleotide comprises at least one homology arm, wherein the homology arm is less than 150 nucleotides in length and comprises sequence homology to a targeted genomic locus of the fungal cell, wherein the Cas endonuclease and guide polynucleotide form a complex that enables the Cas endonuclease to act at or near the targeted genomic locus of the fungal cell, and wherein the donor polynucleotide is a single strand DNA. In certain embodiments, the donor polynucleotide is inserted (incorporated) into the targeted genomic locus of the fungal cell.

In certain other embodiments, the donor polynucleotide further comprises a nucleotide sequence of interest which is either upstream (5') and operably linked to the homology arm or downstream (3') and operably linked to the homology arm. The nucleotide sequence of interest can comprise a single nucleotide, two nucleotides, three nucleotides, *etc.* In other embodiments, a nucleotide sequence of interest is a polynucleotide generally comprising five (5) or more nucleotides. In certain embodiments, the homology arm is between 100-40 nucleotides in length.

In particular embodiments, the nucleotide sequence of interest is inserted (incorporated) into the targeted genomic locus of the fungal cell. In other embodiments, the inserted donor nucleic acid or polynucleotide results in a genome modification selected from the group consisting of a DNA deletion, a DNA disruption, a DNA insertion, a DNA inversion, a DNA point mutation, a DNA replacement, a DNA knock-in, a DNA knock-out and a DNA knock-down.

In yet other embodiments, the donor polynucleotide comprises a homology arm upstream (5') and operably linked to a nucleotide sequence of interest and a homology arm downstream (3') and operably linked to the same nucleotide sequence of interest, wherein at least one of the two homology arms are less than 150 nucleotides in length. In another embodiment, at least one homology arm is between 100-40 nucleotides in length. In other embodiments, both homology arms are less than 500 nucleotides. In certain other embodiments, the nucleotide sequence of interest comprises at least one heterologous nucleotide. In yet other embodiments, the nucleotide sequence of interest comprises a heterologous polynucleotide sequence.

In other embodiments, the Cas endonuclease is a Cas nickase or a functional variant thereof. In particular embodiments, the Cas endonuclease is a Cas9 endonuclease or a functional variant thereof. In other embodiments, the Cas9 endonuclease is a Cas9 endonuclease derived from a genus selected from the group consisting of *Streptococcus sp., Campylobacter sp., Neisseria sp., Francisella sp.* and *Pasteurella sp.*

In other embodiments, the introducing step comprises introducing a polynucleotide construct comprising an expression cassette for expressing the Cas endonuclease (or a functional variant thereof) in the fungal cell. In another embodiment, the introducing step comprises introducing a polynucleotide construct comprising an expression cassette for expressing the guide polynucleotide in the fungal cell. In another embodiment, the introducing step comprises introducing into the fungal cell a circular polynucleotide construct comprising an expression cassette for the Cas endonuclease, an expression cassette for the guide RNA, and the donor DNA. In another embodiment, the introducing step comprises directly introducing the guide polynucleotide or Cas endonuclease into the fungal cell.

In certain embodiment, the Cas endonuclease (or a functional variant thereof) is operably linked to a nuclear localization signal.

In other embodiments, the filamentous fungal cell is selected from the genus consisting of *Trichoderma, Penicillium, Aspergillus, Humicola, Chrysosporium, Fusarium, Myceliophthora, Neurospora* and *Emericella.*

### BRIEF DESCRIPTION OF THE DRAWINGS

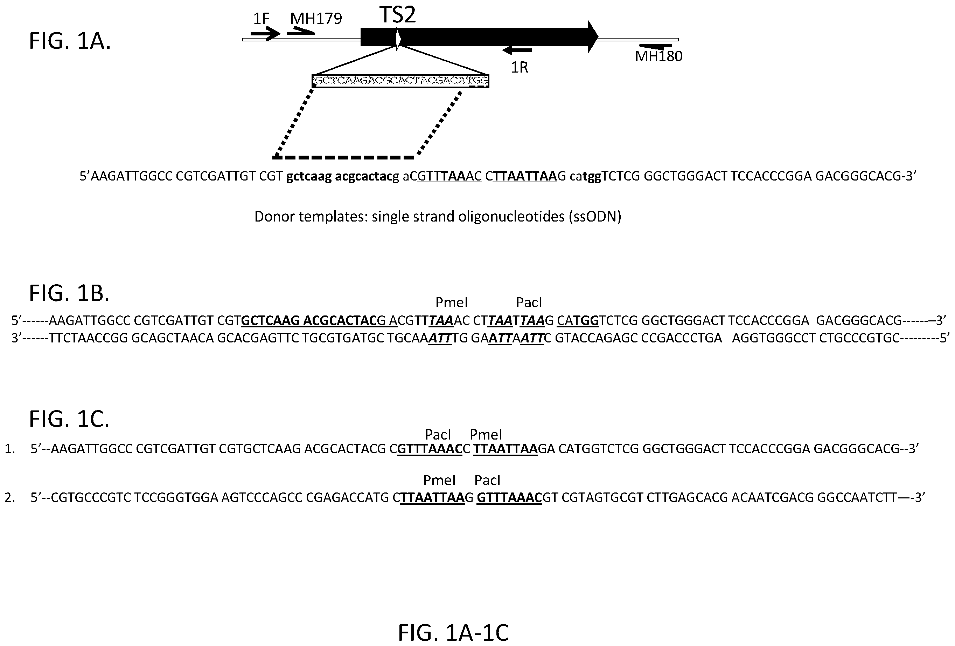

**FIGURES 1A-1C****:** depict a 100-mer single strand donor template with a 19 nucleotide insertion sequence into the *pyr4* locus (TS2). **FIG. 1A****:** The schematic of *pyr4* genomic locus target site 2 (TS2). 1F & 1R, MH179 & 180 are the PCR primers used for analysis. The single strand oligonucleotide of 100 bases is the donor template. **FIG. 1B****:** The 100-nt upper strand of single strand donor DNA. The 19-nt insertion sequence will create 2 restriction sites (*Pme1, Pac1*) and the stop codon (TAA) in 3 different readings frames creating a loss of function mutation in the *pyr4* gene locus. **FIG. 1C****:** The donor templates for homologous recombination: 100 bases single strand oligonucleotides: in **1C-1,** the 100-nt upper strand, and in **1C-2,** the 100-nt lower strand sequences (complementary strand to target site).

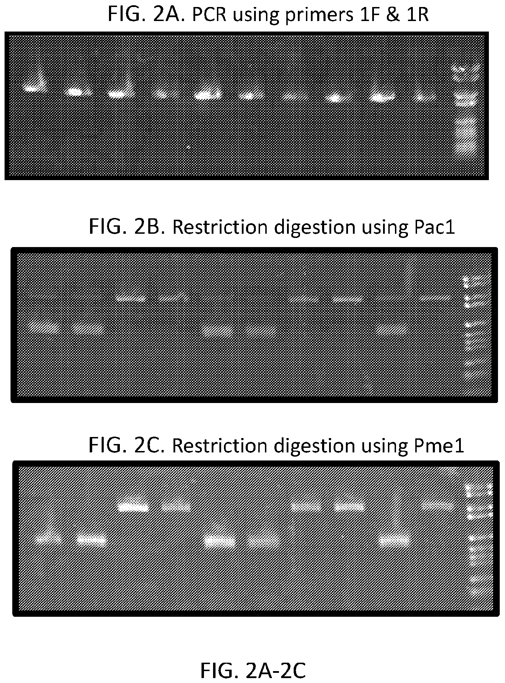

**FIGURES 2A-2C****:** depict homology directed repair using the 100 bases single strand DNA template and efficiency of genome editing. **FIG. 2A****:** PCR amplifications were carried out on DNA extracted from FOA resistant colonies using primers 1F & 1R, SEQ ID NO: 12 & 13. PCR amplifications across the target site TS2 in the *pyr4* gene resulting in 1.2 kb product. **FIG. 2B****:** Restriction digestions of PCR products showed the presence of *Pac1.* **FIG. 2C****:** Restriction digestions of PCR products showed the presence of *Pme1.*

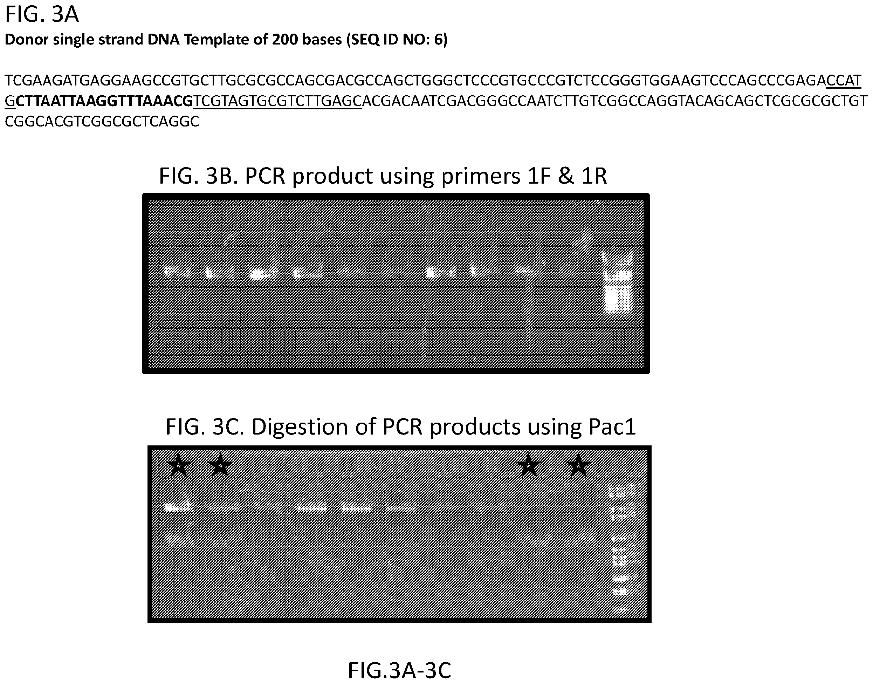

**FIGURES 3A-3C****:** depict homology directed repair using the 200 bases single strand DNA template and efficiency of genome editing. **FIG. 3A****:** Sequence of single strand DNA template of 200 bases. **FIG. 3B****:** PCR amplifications were carried out on DNA extracted from FOA resistant colonies using primers 1F & 1R, SEQ ID NO: 12 & 13. PCR amplifications across the target site TS2 in the *pyr4* gene resulting in 1.2 kb product. **FIG. 3C****:** Restriction digestions of PCR products showed the presence of *Pac1.*

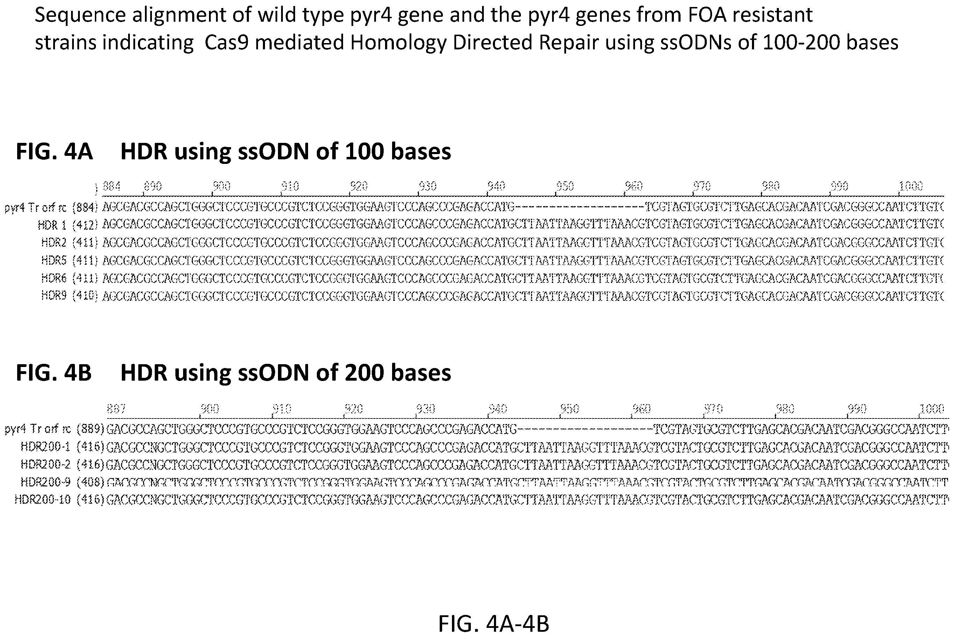

**FIGURE 4****:** depicts sequence alignment of wild type *pyr4* gene and the *pyr4* genes from FOA resistant strains indicating Cas9 mediated Homology Directed Repair using single strand DNA template of 100 (A) and 200 (B) bases.

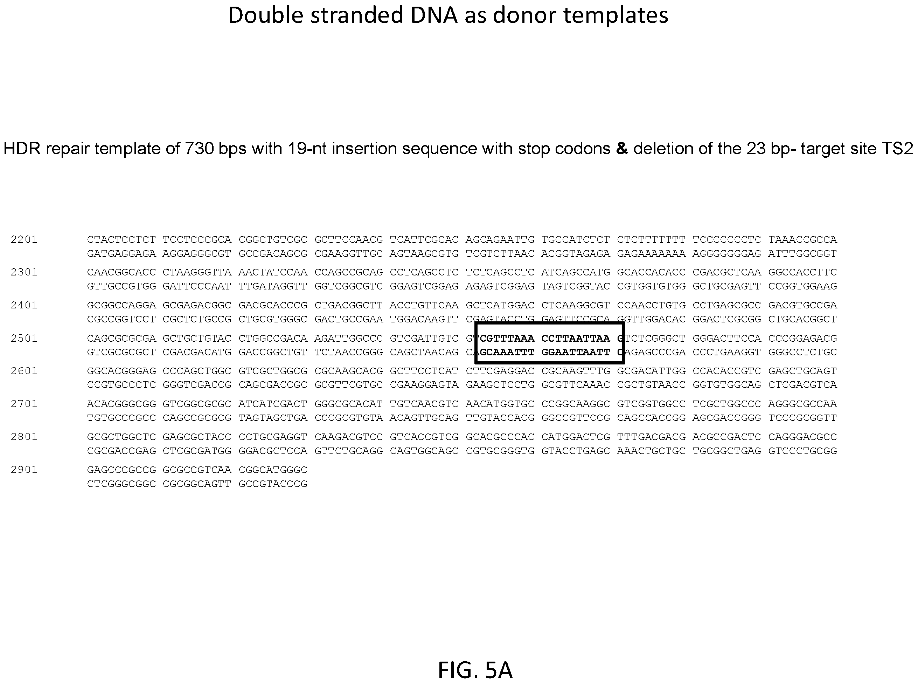

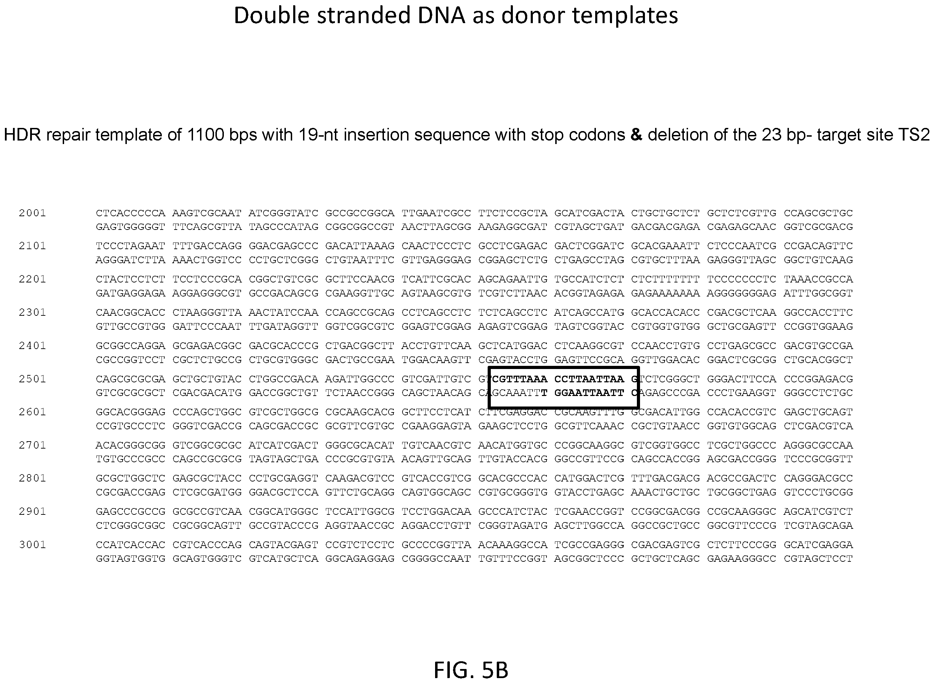

**FIGURE 5****:** depicts the sequences of double strand DNA template with the insertion codons and the flanking homologous arms.

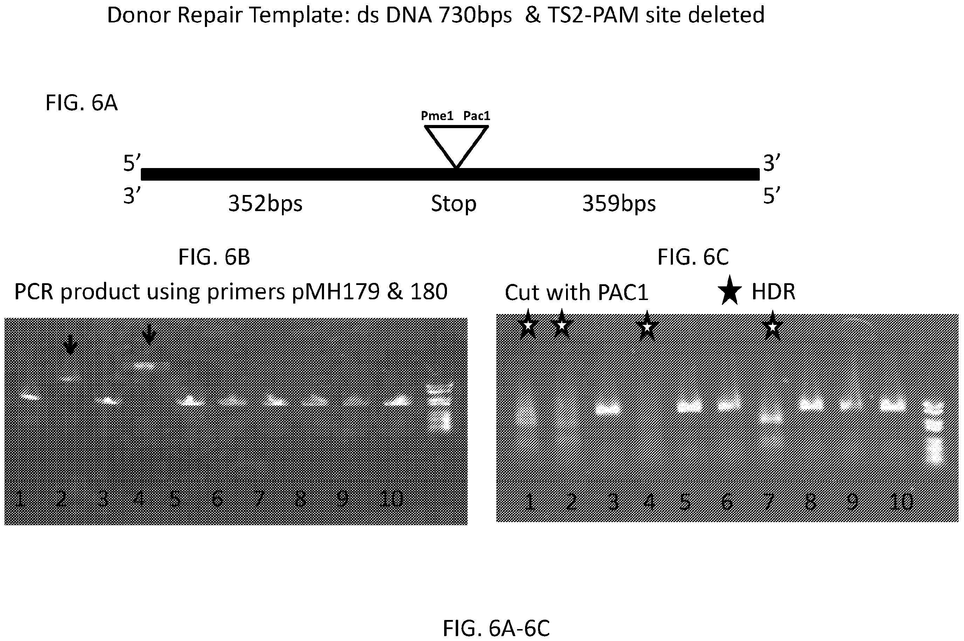

**FIGURES 6A-6C****:** depict double strand DNA donor repair template of length 730 bps. **FIG. 6A****:** Schematic diagram showing the 730 bps double strand DNA. **FIG. 6B****:** PCR amplification of the *pyr4* gene locus across TS2 using primers (SEQ ID NOs:14 & 15) from the genomic DNA of FOA resistant colonies. **FIG. 6C****:** Restriction enzyme digestion of the PCR products by using *Pacl.*

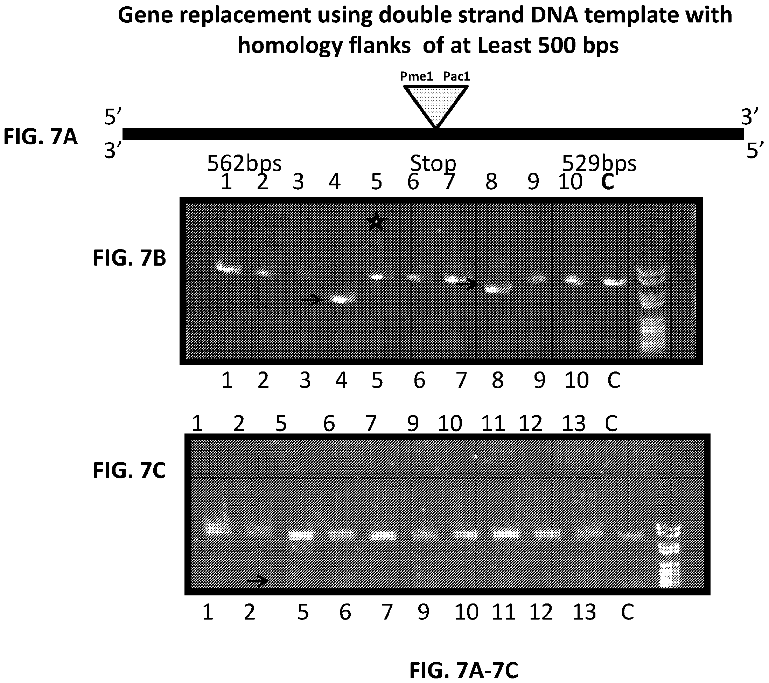

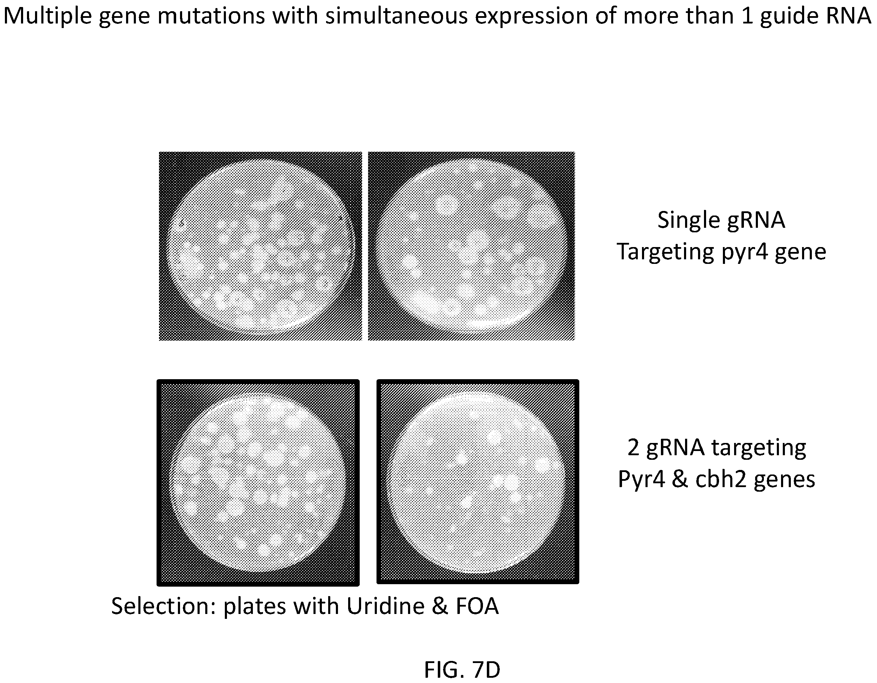

**FIGURES 7A-7C****:** depict double strand DNA donor repair template of length 1100 bps. **FIG. 7A****:** Schematic diagram showing the 1100 bps ds DNA. **FIG. 7B****:** PCR amplification of the *pyr4* gene locus across TS2 using primers (SEQ ID NOs:14 & 15) from the genomic DNA of FOA resistant colonies. **FIG. 7C****:** Restriction enzyme digestion of the PCR products by using *Pacl.*

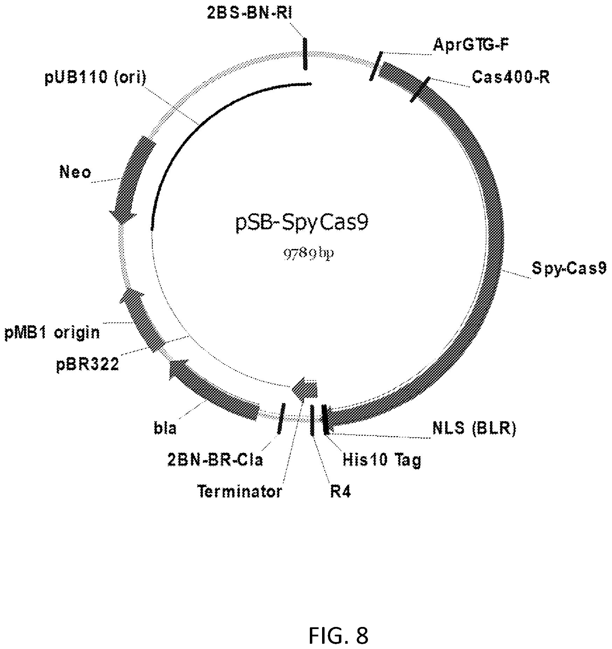

**FIGURE 8****:** depicts the pSB-SpyCas9 expression vector.

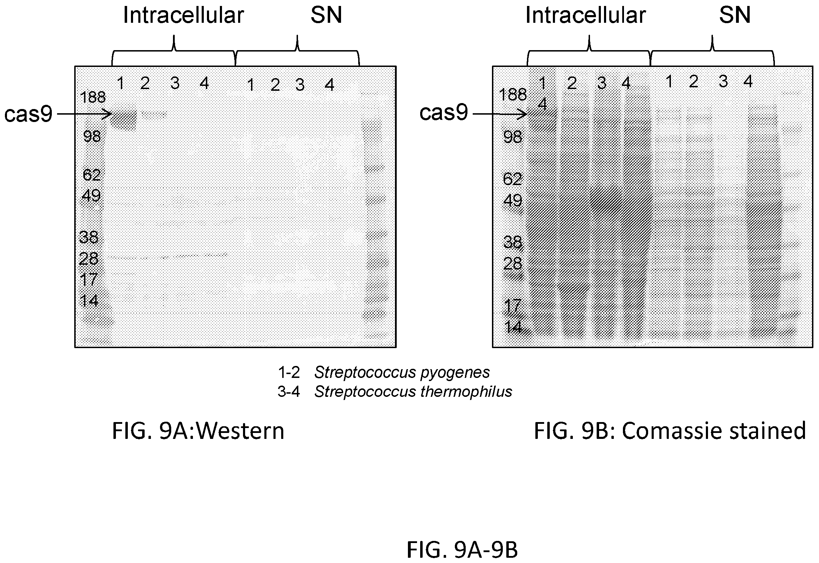

**FIGURES 9A-9B****:** depict the SDS-PAGE analysis of intracellularly expressed Cas9 in *Bacillus sutbilis* showing high levels of production of Cas9. **FIG. 9A** depicts the Western Blot of the SDS-PAGE. **FIG. 9B** depicts the Coomassie stained SDS-PAGE, as *per* Example 8.

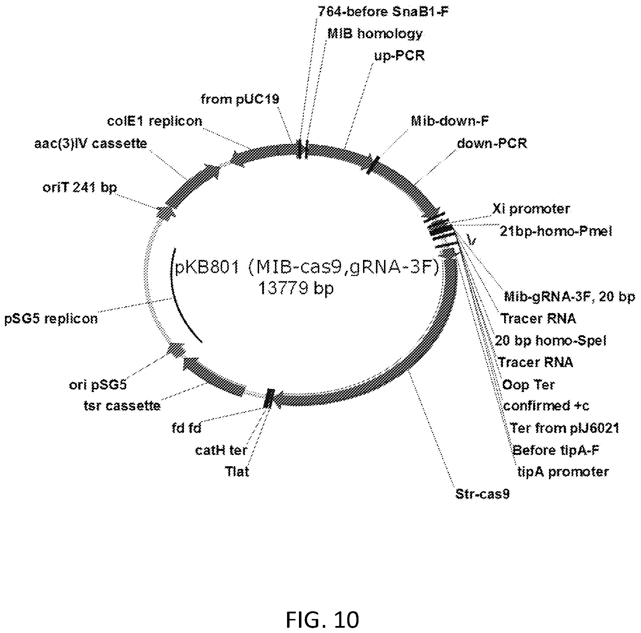

**FIGURE 10** depicts an expression cassette as *per* Example 10, which shows the 2 kb homology arms, the Cas9 gene, and the guide RNA in a single plasmid, used for Cas9-mediated targeted disruption of the *Streptomyces* MIB gene.

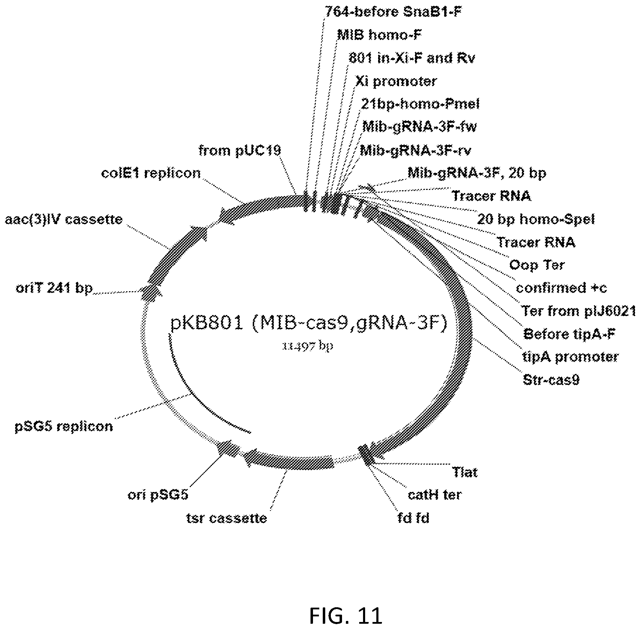

**FIGURE 11** depicts an expression cassette showing the Cas9 gene, the guide RNAs in a single plasmid, but without the 2 kb homology arms, for targeted disruption of the MIB gene. The lack of the homology arms allowed the use of ultramers as donor for homologous recombination as per descriptions of Example 10.

### DETAILED DESCRIPTION

### Overview

Disclosed herein are methods for genome editing in a filamentous fungal cell.

### Abbreviations and Acronyms

The following abbreviations/acronyms have the following meanings unless otherwise specified:
- cDNA: complementary DNA
- DNA: deoxyribonucleic acid
- EDTA: ethylenediaminetetraacetic acid
- kDa: kiloDalton
- MW: molecular weight
- PEG: polyethyleneglycol
- ppm: parts per million, *e.g.*, µg protein per gram dry solid
- RNA: ribonucleic acid
- SDS-PAGE: sodium dodecyl sulfate polyacrylamide gel electrophoresis
- sp.: species
- Tm: melting temperature
- w/v: weight/volume
- w/w: weight/weight
- °C: degrees Centigrade
- H₂O: water
- g or gm: grams
- µg: micrograms
- mg: milligrams
- kg: kilograms
- µL and µl: microliters
- mL and ml: milliliters
- mm: millimeters
- µm: micrometer
- M: molar
- mM: millimolar
- µM: micromolar
- U: units
- sec: seconds
- min(s): minute/minutes
- hr(s): hour/hours
- Tris-HCI: tris(hydroxymethyl)aminomethane hydrochloride
- HEPES: 4-(2-hydroxyethyl)-1-piperazineethanesulfonic acid
- CV: column volumes

### Definitions

Prior to describing the present compositions and methods, the following terms and phrases are defined. Terms not defined herein should be accorded their ordinary meaning as is known and used in the art.

Where a range of values is provided, it is understood that each intervening value, to the tenth of the unit of the lower limit unless the context clearly dictates otherwise, between the upper and lower limit of that range and any other stated or intervening value in that stated range, is encompassed within the present compositions and methods. The upper and lower limits of these smaller ranges may independently be included in the smaller ranges and are also encompassed within the present compositions and methods, subject to any specifically excluded limit in the stated range. Where the stated range includes one or both of the limits, ranges excluding either or both of those included limits are also included in the present compositions and methods.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating un-recited number may be a number which, in the context in which it is presented, provides the substantial equivalent of the specifically recited number. For example, in connection with a numerical value, the term "about" refers to a range of -10% to +10% of the numerical value, unless the term is otherwise specifically defined in context. In another example, the phrase a "pH value of about 6" refers to pH values of from 5.4 to 6.6, unless the pH value is specifically defined otherwise.

The headings provided herein are not limitations of the various aspects or embodiments of the present compositions and methods which can be had by reference to the specification as a whole. Accordingly, the terms defined immediately below are more fully defined by reference to the specification as a whole.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art to which the present compositions and methods belongs. Although any methods and materials similar or equivalent to those described herein can also be used in the practice or testing of the present compositions and methods, representative illustrative methods and materials are now described.

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

It is further noted that the claims may be drafted to "exclude" any optional element. As such, this statement is intended to serve as antecedent basis for use of such exclusive terminology as "solely", "only", "not including", "excluding" and the like in connection with the recitation of claim elements, or use of a "negative" limitation.

It is further noted that the term "consisting essentially of," as used herein refers to a composition wherein the component(s) after the term is in the presence of other known component(s) in a total amount that is less than 30% by weight of the total composition and do not contribute to or interferes with the actions or activities of the component(s).

It is further noted that the term "comprising", as used herein, means including, but not limited to, the component(s) after the term "comprising." The component(s) after the term "comprising" are required or mandatory, but the composition comprising the component(s) may further include other non-mandatory or optional component(s).

It is also noted that the term "consisting of," as used herein, means including, and limited to, the component(s) after the term "consisting of." The component(s) after the term "consisting of" are therefore required or mandatory, and no other component(s) are present in the composition.

As used herein, a polypeptide referred to as a "Cas endonuclease" or having "Cas endonuclease activity" relates to a CRISPR associated (Cas) polypeptide encoded by a Cas gene, wherein the Cas polypeptide is capable of cutting a target DNA sequence when functionally coupled with one or more guide polynucleotides (*see, e.g.,* U.S. Patent 8,697,359). Variants of Cas endonucleases that retain guide polynucleotide directed endonuclease activity are also included in this definition. The Cas endonucleases employed in the donor DNA insertion methods detailed herein are endonucleases that introduce double-strand breaks into the DNA at the target site. A Cas endonuclease is guided by the guide polynucleotide to recognize and cleave a specific target site in double stranded DNA (*e.g.,* at a target site in the genome of a cell).

As used herein, the term "genome-editing" is a type of genetic engineering in which DNA is inserted, replaced, or removed from a genome using artificially engineered nucleases, or "molecular scissors." It is a useful tool to elucidate the function and effect of a gene or protein in a sequence specific manner.

As used herein, the term "guide polynucleotide" relates to a polynucleotide sequence that can form a complex with a Cas endonuclease and enables the Cas endonuclease to recognize and cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited to, Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA".

The guide polynucleotide can be a double molecule (also referred to as duplex guide polynucleotide) comprising a first nucleotide sequence domain (referred to as Variable Targeting domain or VT domain) that is complementary to a nucleotide sequence in a target DNA and a second nucleotide sequence domain (referred to as Cas endonuclease recognition domain or CER domain) that interacts with a Cas endonuclease polypeptide. The CER domain of the double molecule guide polynucleotide comprises two separate molecules that are hybridized along a region of complementarity. The two separate molecules can be RNA, DNA, and/or RNA-DNA- combination sequences. In some embodiments, the first molecule of the duplex guide polynucleotide comprising a VT domain linked to a CER domain is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the crRNA naturally occurring in Bacteria and Archaea. In one embodiment, the size of the fragment of the crRNA naturally occurring in Bacteria and Archaea that is present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some embodiments the second molecule of the duplex guide polynucleotide comprising a CER domain is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides). In certain embodiments, the RNA that guides the RNA/Cas9 endonuclease complex is a duplexed RNA comprising a duplex crRNA-tracrRNA.

The guide polynucleotide can also be a single molecule comprising a first nucleotide sequence domain (referred to as Variable Targeting domain or VT domain) that is complementary to a nucleotide sequence in a target DNA and a second nucleotide domain (referred to as Cas endonuclease recognition domain or CER domain) that interacts with a Cas endonuclease polypeptide. By "domain" it is meant a contiguous stretch of nucleotides that can be RNA, DNA, and/or RNA-DNA-combination sequence. The VT domain and / or the CER domain of a single guide polynucleotide can comprise a RNA sequence, a DNA sequence, or a RNA-DNA-combination sequence. In some embodiments the single guide polynucleotide comprises a crNucleotide (comprising a VT domain linked to a CER domain) linked to a tracrNucleotide (comprising a CER domain), wherein the linkage is a nucleotide sequence comprising a RNA sequence, a DNA sequence, or a RNA-DNA combination sequence. The single guide polynucleotide being comprised of sequences from the crNucleotide and tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). In one embodiment of the disclosure, the single guide RNA comprises a crRNA or crRNA fragment and a tracrRNA or tracrRNA fragment of the type II CRISPR/Cas system that can form a complex with a type II Cas endonuclease, wherein the guide RNA/Cas endonuclease complex can direct the Cas endonuclease to a fungal cell genomic target site, enabling the Cas endonuclease to introduce a double strand break into the genomic target site.

One aspect of using a single guide polynucleotide versus a duplex guide polynucleotide is that only one expression cassette needs to be made to express the single guide polynucleotide in a target cell.

The term "Cas endonuclease recognition domain" or "CER domain" of a guide polynucleotide is used interchangeably herein and includes a nucleotide sequence (such as a second nucleotide sequence domain of a guide polynucleotide), that interacts with a Cas endonuclease polypeptide. The CER domain can be composed of a DNA sequence, a RNA sequence, a modified DNA sequence, a modified RNA sequence (see for example modifications described herein), or any combination thereof.

The nucleotide sequence linking the crNucleotide and the tracrNucleotide of a single guide polynucleotide can comprise a RNA sequence, a DNA sequence, or a RNA-DNA combination sequence. In one embodiment, the nucleotide sequence linking the crNucleotide and the tracrNucleotide of a single guide polynucleotide can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In another embodiment, the nucleotide sequence linking the crNucleotide and the tracrNucleotide of a single guide polynucleotide can comprise a tetra-loop sequence, such as, but not limited to, a GAAA tetra-loop sequence.

Nucleotide sequence modification of the guide polynucleotide, and/or CER domain can be selected from, but not limited to, the group consisting of a 5' cap, a 3' polyadenylated tail, a riboswitch sequence, a stability control sequence, a sequence that forms a dsRNA duplex, a modification or sequence that targets the guide poly nucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

As used herein, the term "guide polynucleotide/Cas endonuclease system" (and equivalents) includes a complex of a Cas endonuclease and a guide polynucleotide (single or double) that is capable of introducing a double strand break into a DNA target sequence. The Cas endonuclease unwinds the DNA duplex in close proximity of the genomic target site and cleaves both DNA strands upon recognition of a target sequence by a guide RNA, but only if the correct protospacer-adjacent motif (PAM) is appropriately oriented at the 3' end of the target sequence.

As used herein, the terms "functional fragment", "fragment that is functionally equivalent", "functionally equivalent fragment", and the like, are used interchangeably and refer to a portion or subsequence of a parent polypeptide that retains the qualitative enzymatic activity of the parent polypeptide. For example, a functional fragment of a Cas endonuclease retains the ability to create a double-strand break with a guide polynucleotide. It is noted here that a functional fragment may have altered quantitative enzymatic activity as compared to the parent polypeptide.

Relatedly, the terms "functional variant", "variant that is functionally equivalent", "functionally equivalent variant", and the like are used interchangeably and refer to a variant of a parent polypeptide that retains the qualitative enzymatic activity of the parent polypeptide. For example, a functional variant of a Cas endonuclease retains the ability to create a double-strand break with a guide polynucleotide. It is noted here that a functional variant may have altered quantitative enzymatic activity as compared to the parent polypeptide.

Fragments and variants can be obtained *via* any convenient method, including site-directed mutagenesis and synthetic construction.

As used herein, the term "codon-modified gene" or "codon-preferred gene" or "codon-optimized gene" is a gene having its frequency of codon usage designed to mimic the frequency of preferred codon usage of the host cell. The nucleic acid changes made to codon-optimize a gene are "synonymous", meaning that they do not alter the amino acid sequence of the encoded polypeptide of the parent gene. However, both native and variant genes can be codon-optimized for a particular host cell, and as such no limitation in this regard is intended.

As used herein, the term "coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to: promoters, translation leader sequences, 5' untranslated sequences, 3' untranslated sequences, introns, polyadenylation target sequences, RNA processing sites, effector binding sites, and stem-loop structures.

As used herein, the term "promoter" refers to a DNA sequence capable of controlling the expression of a coding sequence or functional RNA. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. As is well-known in the art, promoters can be categorized according to their strength and/or the conditions under which they are active, *e.g.*, constitutive promoters, strong promoters, weak promoters, inducible/repressible promoters, tissue-specific/developmentally regulated promoters, cell-cycle dependent promoters, *etc.*

As used herein, the term "RNA transcript" refers to the product resulting from RNA polymerase-catalyzed transcription of a DNA sequence. "Messenger RNA" or "mRNA" refers to the RNA that is without introns and that can be translated into protein by the cell. "cDNA" refers to a DNA that is complementary to, and synthesized from, a mRNA template using the enzyme reverse transcriptase. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that, under certain conditions, blocks the expression of a target gene (*see, e.g.,* U.S. Patent No. 5,107,065). The complementarity of an antisense RNA may be with any part of the specific gene transcript, *i.e.,* at the 5' non-coding sequence, 3' non-coding sequence, introns, or the coding sequence. "Functional RNA" refers to antisense RNA, ribozyme RNA, or other RNA that may not be translated into a polypeptide but yet has an effect on cellular processes. The terms "complement" and "reverse complement" are used interchangeably herein with respect to mRNA transcripts, and are meant to define the antisense RNA of the message.

As used herein, the term "functionally attached" or "operably linked" means that a regulatory region or functional domain of a polypeptide or polynucleotide sequence having a known or desired activity, such as a promoter, enhancer region, terminator, signal sequence, epitope tag, *etc.,* is attached to or linked to a target (*e.g.,* a gene or polypeptide) in such a manner as to allow the regulatory region or functional domain to control the expression, secretion or function of that target according to its known or desired activity. For example, a promoter is operably linked with a coding sequence when it is capable of regulating the expression of that coding sequence (*i.e.,* the coding sequence is under the transcriptional control of the promoter).

As used herein, the term "PCR" or "polymerase chain reaction" is a technique for the synthesis of specific DNA segments and consists of a series of repetitive denaturation, annealing, and extension cycles and is well known in the art.

As used herein, the term "recombinant," when used in reference to a biological component or composition (*e.g.,* a cell, nucleic acid, polypeptide/enzyme, vector, *etc.*) indicates that the biological component or composition is in a state that is not found in nature. In other words, the biological component or composition has been modified by human intervention from its natural state. For example, a recombinant cell encompasses a cell that expresses one or more genes that are not found in its native parent (*i.e.,* non-recombinant) cell, a cell that expresses one or more native genes in an amount that is different than its native parent cell, and/or a cell that expresses one or more native genes under different conditions than its native parent cell. Recombinant nucleic acids may differ from a native sequence by one or more nucleotides, be operably linked to heterologous sequences (*e.g.*, a heterologous promoter, a sequence encoding a non-native or variant signal sequence, *etc.*)*,* be devoid of intronic sequences, and/or be in an isolated form. Recombinant polypeptides/enzymes may differ from a native sequence by one or more amino acids, may be fused with heterologous sequences, may be truncated or have internal deletions of amino acids, may be expressed in a manner not found in a native cell (*e.g.*, from a recombinant cell that over-expresses the polypeptide due to the presence in the cell of an expression vector encoding the polypeptide), and/or be in an isolated form. It is emphasized that in some embodiments, a recombinant polynucleotide or polypeptide/enzyme has a sequence that is identical to its wild-type counterpart but is in a non-native form (*e.g.*, in an isolated or enriched form).

As used herein, the terms "plasmid", "vector" and "cassette" refer to an extra chromosomal element that carries a polynucleotide sequence of interest, *e.g.*, a gene of interest to be expressed in a cell (an "expression vector" or "expression cassette"). Such elements are generally in the form of double-stranded DNA and may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. The polynucleotide sequence of interest may be a gene encoding a polypeptide or functional RNA that is to be expressed in the target cell. Expression cassettes/vectors generally contain a gene with operably linked elements that allow for expression of that gene in a host cell.

As used herein, the term "expression", as used herein, refers to the production of a functional end-product (*e.g.*, an mRNA, guide RNA, or a protein) in either precursor or mature form.

As used herein, the term "introduced" in the context of inserting a polynucleotide or polypeptide into a cell (*e.g.,* a recombinant DNA construct/expression construct) refers to any method for performing such a task, and includes any means of "transfection", "transformation", "transduction", physical means, or the like, to achieve introduction of the desired biomolecule.

By "introduced transiently", "transiently introduced", "transient introduction", "transiently express" and the like is meant that a biomolecule is introduced into a host cell (or a population of host cells) in a non-permanent manner. With respect to double stranded DNA, transient introduction includes situations in which the introduced DNA does not integrate into the chromosome of the host cell and thus is not transmitted to all daughter cells during growth as well as situations in which an introduced DNA molecule that may have integrated into the chromosome is removed at a desired time using any convenient method (*e.g.*, employing a cre-Iox system, by removing positive selective pressure for an episomal DNA construct, by promoting looping out of all or part of the integrated polynucleotide from the chromosome using a selection media, *etc.*)*.* No limitation in this regard is intended. In general, introduction of RNA (*e.g.,* a guide RNA, a messenger RNA, ribozyme, *etc*.) or a polypeptide (*e.g.*, a Cas polypeptide) into host cells is considered transient in that these biomolecules are not replicated and indefinitely passed down to daughter cells during cell growth. With respect to the Cas/guide RNA complex, transient introduction covers situations when either of the components is introduced transiently, as both biomolecules are needed to exert targeted Cas endonuclease activity. Thus, transient introduction of a Cas/guide RNA complexes includes embodiments where either one or both of the Cas endonuclease and the guide RNA are introduced transiently. For example, a host cell having a genome-integrated expression cassette for the Cas endonuclease (and thus not transiently introduced) into which a guide RNA is transiently introduced can be said to have a transiently introduced Cas/guide RNA complex (or system) because the functional complex is present in the host cell in a transient manner.

As used herein, the term "mature" protein refers to a post-translationally processed polypeptide (*i.e.,* one from which any pre- or pro-peptides present in the primary translation product have been removed). "Precursor" protein refers to the primary product of translation of mRNA (*i.e.,* with pre- and pro-peptides still present). Pre- and pro-peptides may be, but are not limited to, intracellular localization signals.

As used herein, the term "fungal cell", "fungi", "fungal host cell", and the like, as used herein includes the phyla *Ascomycota, Basidiomycota, Chytridiomycota,* and *Zygomycota* (as defined by Hawksworth *et al.,* 1995), as well as the *Oomycota* (Hawksworth *et al*.,1995) and all mitosporic fungi (Hawksworth *et al*. ,1995). In certain embodiments, the fungal host cell is a yeast cell, wherein the term "yeast" is meant ascosporogenous yeast (Endomycetales), basidiosporogenous yeast, and yeast belonging to the *Fungi Imperfecti* (Blastomycetes). As such, a yeast host cell includes a *Candida, Hansenula, Kluyveromyces, Pichia, Saccharomyces, Schizosaccharomyces,* or *Yarrowia* cell. Species of yeast include, but are not limited to, *Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Kluyveromyces lactis,* and *Yarrowia lipolytica.*

As used herein, the term "filamentous fungal cell" includes all filamentous forms of the subdivision *Eumycotina.* Suitable cells of filamentous fungal genera include, but are not limited to, cells of *Acremonium, Aspergillus, Aureobasidium, Bjerkandera, Ceriporiopsis, Chrysoporium, Coprinus, Coriolus, Corynascus, Chaertomium, Cryptococcus, Filobasidium, Fusarium, Gibberella, Humicola, Magnaporthe, Mucor, Myceliophthora, Mucor, Neocallimastix, Neurospora, Paecilomyces, Penicillium, Phanerochaete, Phlebia, Piromyces, Pleurotus, Scytaldium, Schizophyllum, Sporotrichum, Talaromyces, Thermoascus, Thielavia, Tolypocladium, Trametes,* and *Trichoderma.*

Suitable cells of filamentous fungal species include, but are not limited to, cells of *Aspergillus awamori, Aspergillus fumigatus, Aspergillus foetidus, Aspergillus japonicus, Aspergillus nidulans, Aspergillus niger, Aspergillus oryzae, Chrysosporium lucknowense, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Bjerkandera adusta, Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Coprinus cinereus, Coriolus hirsutus, Humicola insolens, Humicola lanuginosa, Mucor miehei, Myceliophthora thermophila, Neurospora crassa, Neurospora intermedia, Penicillium purpurogenum, Penicillium canescens, Penicillium solitum, Penicillium funiculosum Phanerochaete chrysosporium, Phlebia radiate, Pleurotus eryngii, Talaromyces flavus, Thielavia terrestris, Trametes villosa, Trametes versicolor, Trichoderma harzianum, Trichoderma koningii, Trichoderma longibrachiatum, Trichoderma reesei,* and *Trichoderma viride.*

As used herein, the terms "target site", "target sequence", "genomic target site", "genomic target sequence" (and equivalents thereof) are used interchangeably herein and refer to a polynucleotide sequence in the genome of a fungal cell at which a Cas endonuclease cleavage is desired to promote a genome modification, *e.g.*, insertion of a donor DNA and subsequent deletion of a genomic region of interest. The context in which this term is used, however, can slightly alter its meaning. For example, the target site for a Cas endonuclease is generally very specific and can often be defined to the exact nucleotide position, whereas in some cases the target site for a desired genome modification can be defined more broadly than merely the site at which DNA cleavage occurs, *e.g.*, a genomic locus or region that is to be deleted from the genome. Thus, in certain cases, the genome modification that occurs *via* the activity of Cas/guide RNA DNA cleavage is described as occurring "at or near" the target site. The target site can be an endogenous site in the fungal cell genome, or alternatively, the target site can be heterologous to the fungal cell and thereby not be naturally occurring in the genome, or the target site can be found in a heterologous genomic location compared to where it occurs in nature.

As used herein, the term "nucleic acid" means a polynucleotide and includes a single or a double-stranded polymer of deoxyribonucleotide or ribonucleotide bases. Nucleic acids may also include fragments and/or modified nucleotides. Thus, the terms "polynucleotide", "nucleic acid sequence", "nucleotide sequence" and "nucleic acid fragment" are used interchangeably to denote a polymer of RNA and/or DNA that is single-stranded or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

As used herein, the term "derived from" encompasses the terms "originated from," "obtained from," "obtainable from," "isolated from," and "created from," and generally indicates that one specified material find its origin in another specified material or has features that can be described with reference to the another specified material.

As used herein, the term "substantially similar" or "substantially identical," in the context of at least two nucleic acids or polypeptides, means that a polynucleotide or polypeptide comprises a sequence that has at least 90%, at least 91%, at least 92%, at least 93%, at least 94%, at least 95%, at least 96%, at least 97%, at least 98%, or even at least 99% identical to a parent or reference sequence, or does not include amino acid substitutions, insertions, deletions, or modifications made only to circumvent the present description without adding functionality.

As used herein, the term "sequence identity" or "identity" in the context of nucleic acid or polypeptide sequences refers to the nucleic acid bases or amino acid residues in two sequences that are the same when aligned for maximum correspondence over a specified comparison window.

As used herein, the term "percentage of sequence identity" refers to the value determined by comparing two optimally aligned sequences over a comparison window, wherein the portion of the polynucleotide or polypeptide sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

As used herein, the term "Clustal V method of alignment" corresponds to the alignment method labeled Clustal V (Higgins and Sharp, 1989; Higgins *et al.,* 1992) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

As used herein, the term "Clustal W method of alignment" corresponds to the alignment method labeled Clustal W (Higgins and Sharp, 1989; Higgins *et al.,* 1992) and found in the MegAlign™ v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, 1989). GAP uses the algorithm of Needleman and Wunsch, (1970), to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

As used herein, the term "gene" includes a nucleic acid fragment that encodes and is capable to express a functional molecule such as, but not limited to, a specific polypeptide (*e.g.,* an enzyme) or a functional RNA molecule (*e.g.,* a guide RNA, an anti-sense RNA, ribozyme, *etc.*)*,* and includes regulatory sequences preceding (5' non-coding sequences) and/or following (3' non-coding sequences) the coding sequence. "Native gene" refers to a gene as found in nature with its own regulatory sequences.

As used herein, the term "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, a mutated gene comprises an alteration that results from a guide polynucleotide/Cas endonuclease system as disclosed herein. A mutated fungal cell is a fungal cell comprising a mutated gene.

As used herein, the term "targeted mutation" is a mutation in a native gene that was made by altering a target sequence within the native gene using a method involving a double-strand-break-inducing agent that is capable of inducing a double-strand break in the DNA of the target sequence as disclosed herein or known to one skilled in the art.

The term "polynucleotide modification template" refers to a polynucleotide that comprises at least one nucleotide modification when compared to the nucleotide sequence to be edited. A nucleotide modification can include, for example: (i) a replacement of at least one nucleotide, (ii) a deletion of at least one nucleotide, (iii) an insertion of at least one nucleotide, or (iv) any combination of (i)-(iii). Optionally, the polynucleotide modification template can further comprise homologous nucleotide sequences flanking the at least one nucleotide modification, wherein the flanking homologous nucleotide sequences provide sufficient homology to the desired nucleotide sequence to be edited. The flanking homologous sequences are alternatively referred to herein as "homology arms".

As used herein, the terms "donor DNA", "donor nucleic acid sequence" and "donor polynucleotide" refer to a polynucleotide modification template comprising a "polynucleotide of interest" to be inserted into the target site of the Cas endonuclease (*i.e.,* in conjunction with the activity of a Cas endonuclease/guide polynucleotide complex). In certain embodiments, the donor DNA construct comprises at least one region of homology (a "homology arm") that flanks the polynucleotide of interest (*i.e.,* the homology arm is upstream (5') or downstream (3') of the polynucleotide of interest). Thus, in certain embodiments, a donor DNA construct comprising at least one homology arm shares homology to a genomic region present in or flanking the (Cas) target site of the fungal cell genome. In other embodiments, a donor DNA construct comprises both an upstream (5') homology arm and a downstream (3') homology arm flanking the polynucleotide of interest. Thus, in other embodiments of the disclosure, a donor DNA construct comprises a first homology arm which is upstream (5') and operably linked to the polynucleotide of interest and a second homology arm which is downstream (3') and operably linked to the polynucleotide of interest. The first homology arm and second homology arm of the donor DNA construct share homology to a first genomic region and a second genomic region of the (Cas) target site of the fungal cell genome, respectively. Thus, in particular embodiments, a donor DNA (or polynucleotide modification template) comprises two homologous sequences (*i.e.,* 5' and 3' homology arms) separated by a polynucleotide sequence of interest (or a base pair of interest) that is heterologous to the sequence at the target site. Homologous recombination between the genomic target site and the two donor DNA homology arms typically results in the editing of the sequence at the target site.

By "homologous" is meant DNA sequences that are similar. For example, a "region homologous to a genomic sequence" that is found on the donor DNA is a region of DNA that has a similar sequence to a given "genomic sequence" in the fungal cell genome. Collectively, the sequence homologous to a genomic sequence in the genomic locus and the genomic sequence itself are sometimes referred to herein as "the repeat sequences". A homologous region can be of any length that is sufficient to promote looping-out of the loop-out target region *via* homologous recombination between the repeat sequence and the homologous genomic sequence (which can be selected for under selective culture conditions).

For example, the repeat sequence (homology arm) can comprise at least 50-55, 50-60, 50-65, 50-70, 50-75, 50-80, 50-85, 50-90, 50-95, 50-100 bases in length. "Sufficient homology" indicates that two polynucleotide sequences (*e.g.*, direct repeat sequences in the donor DNA and the genome of fungal cell) have sufficient structural similarity to loop-out the sequence in between the repeat sequences, *e.g.*, under appropriate selective culture conditions. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of at least one of the sequences.

As used herein, the term "genomic region" or "genomic locus" is a segment of a chromosome in the genome of a fungal cell that is present on either side of the target site (*e.g.*, including the genomic deletion target and the genomic repeat sequence that is homologous to the repeat sequence in a donor DNA) or, alternatively, also comprises a portion of the target site. The genomic region can comprise at least 50-55, 50-60, 50-65, 50-70, 50-75, 50-80, 50-85, 50-90, 50-95, 50-100, 50-200, 50-300, 50-400, 50-500, 50-600, 50-700, 50-800, 50-900, 50-1000, 50-1100, 50-1200, 50-1300, 50-1400, 50-1500, 50-1600, 50-1700, 50-1800, 50-1900, 50-2000, 50-2100, 50-2200, 50-2300, 50-2400, 50-2500, 50-2600, 50-2700, 50-2800, 50-2900, 50-3000, 50-3100 or more bases.

As used herein, the term "genomic deletion target" and equivalents is the sequence in the fungal genome that a user wants to delete according to aspects of the present disclosure (*e.g., see* FIG. 1). A "loop-out target region" and equivalents is the region between direct repeats (*e.g.*, the genomic repeat sequence and the repeat sequence in the donor DNA that is homologous to the genomic repeat sequence) that is looped-out by homologous recombination between the direct repeats in the fungal genome. In certain embodiments, the loop-out target region includes the genomic deletion target and the selectable marker on the donor DNA inserted at the target site in the fugal genome. A phenotypic marker is a screenable or selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

Examples of selectable markers include, but are not limited to, DNA segments that comprise restriction enzyme sites; DNA segments that encode products which provide resistance against otherwise toxic compounds and antibiotics, such as, chlorimuron ethyl, benomyl, Basta, and hygromycin phosphotransferase (HPT); DNA segments that encode products which are otherwise lacking in the recipient cell (*e.g.*, tRNA genes, auxotrophic markers, dominant heterologous marker-amdS); DNA segments that encode products which can be readily identified (*e.g.*, phenotypic markers such as β-gaiactosidase, GUS; fluorescent proteins such as green fluorescent protein (GFP), cyan (CFP), yellow (YFP), red (RFP), and cell surface proteins); the generation of new primer sites for PCR (*e.g.*, the juxtaposition of two DNA sequence not previously juxtaposed), the inclusion of DNA sequences not acted upon or acted upon by a restriction endonuclease or other DNA modifying enzyme, chemical, *etc.* and, the inclusion of a DNA sequences required for a specific modification (*e.g.*, methylation) that allows its identification.

As used herein, the term "signal sequence" is a sequence of amino acids attached to the N-terminal portion of a protein, which facilitates the secretion of the protein outside the cell. The mature form of an extracellular protein lacks the signal sequence, which is cleaved off during the secretion process.

As used herein, the terms "polypeptide" and "protein" are used interchangeably to refer to polymers of any length comprising amino acid residues linked by peptide bonds. The conventional one-letter or three-letter codes for amino acid residues are used herein. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, etc.), as well as other modifications known in the art.

A "heterologous" nucleic acid construct or sequence has a portion of the sequence which is not native or existing in a native form to the cell in which it is expressed. Heterologous, with respect to a control sequence refers to a control sequence (*i.e.* promoter or enhancer) that does not function in nature to regulate the same gene the expression of which it is currently regulating. Generally, heterologous nucleic acid sequences are not endogenous to the cell or part of the genome in which they are present in the native state, and have been added to the cell, by infection, transfection, transformation, microinjection, electroporation, or the like. A "heterologous" nucleic acid construct may contain a control sequence/DNA coding sequence combination that is the same as, or different from a control sequence/DNA coding sequence combination found in the native cell.

As used herein, the term "host cell", includes any fungus, whether a unicellular organism, a cell derived from a multicellular organism and placed in tissue culture, or a cell present as part of a multicellular organism, which is susceptible to transformation with a nucleic acid construct according to the disclosure. Such host cells, such as yeast and other fungal cells, or bacteria may be used for replicating DNA and producing polypeptides encoded by nucleotide sequences as used in the disclosure. Suitable cells for the present invention are generally filamentous fungi or yeasts. Particularly preferred are cells from filamentous fungi, preferably *Aspergillus,* such as *A. niger* and *A. tubingensis.* Other preferred organisms include any one of *Aspergillus oryzae, A. awamori, Trichoderma reesei, Trichoderma viride* and *Trichoderma longibrachiatum.*

As used herein, the term "introduced" in the context of inserting a nucleic acid sequence into a cell, means "transfection", "transformation" or "transduction," as known in the art.

As used herein, "transformed" means a cell has been transformed by use of recombinant DNA techniques. Transformation typically occurs by insertion of one or more nucleotide sequences into a cell. The inserted nucleotide sequence may be a heterologous nucleotide sequence, *i.e.,* is a sequence that is not natural to the cell that is to be transformed, such as a fusion protein.

As used herein, the term "expression" refers to the process by which a polypeptide is produced based on a nucleic acid sequence. The process includes both transcription and translation.

### Methods for Modifying a Microbial Cell Genome

The present disclosure pertains to methods for genome editing in a microbial cell.

### 1. Methods of Gene Editing

In certain embodiments, fungi of the disclosure are biotechnologically applied microbes used for the production of proteins including different hydrolytic enzymes such as cellulases and xylanases. For example, *Hypocrea jecorina* (synonym *Trichoderma reesei*) is arguably the best studied cellulolytic fungus, and its cellulases and hemicellulases are currently at the forefront of investigation for the enzymatic conversion of renewable lignocellulosic biomass to biofuels. Therefore, there is a need to develop efficient molecular tools to further improve industrial protein/cellulase production and to obtain new insights regarding the mechanism for cellulase or hemicellulase gene regulation.

Gene targeting by homologous recombination (HR) is a key technique to study the function of genes and to alter the characteristics of fungal strains for different applications. Nevertheless, in contrast to the yeast *Saccharomyces cerevisiae* which has a high rate of homologous gene (HR) targeting, the non-homologous end joining pathway (NHEJ) (Bleuyard *et al.,* 2006) pathway seems to be the dominant mode of DNA integration in other fungi, including various filamentous fungi, leading to direct ligation of strands without sequence homology.

HR for gene targeting has been shown to be enhanced when the targeted DNA site contains a double-strand break (*see, e.g.,* Rudin *et al.,* 1989; Smith *et al*.). The type II clustered regularly interspaced short palindromic repeats (CRISPR)/CRISPR-associated gene (Cas) system, the most popular genome-editing tool at this time, can catalyze a double-strand break (DSB) in the target DNA composed of a 20-bp sequence matching the protospacer of the guide RNA (gRNA) and an adjacent downstream 5'-NGG nucleotide sequence (termed as the protospacer-adjacent motif (PAM)) (*see, e.g.,* Cong *et al.,* 2013).

Over the past 2 years, many studies have demonstrated that the CRISPR/Cas9 system is a powerful genome editing method that facilitates genetic alterations in genomes in a variety of organisms. Although Cas-based genome engineering technologies have been applied to a number of different host cell types, even in filamentous fungal cells (Liu *et al.,* 2015), they have limitations including, for example, the gene editing is cas9 expressed microbial strain dependent, and multiple steps of molecular manipulation are needed for donor DNA construction, *etc.*

Thus, based on the foregoing, there remains a need in the art for developing more robustly effective and efficient Cas-based genome editing methods and compositions thereof for modifying/altering a genomic target site in a microbial cell.

### 2. Short Homology Arms in Donor DNA required for Gene Editing

Methods are provided herein employing a guide RNA/Cas endonuclease system for inserting a donor DNA with one or more short homology arms at a target site in the genome of a microbial cell (a filamentous fungal cell), as set out in the claims.

Introducing a Cas endonuclease/guide polynucleotide complex into the cell along with a donor DNA is typically necessary for generating a precise repair of the double strand break in the polynucleotide at the target site in the genome of the microbial cell. The components of the Cas system as provided herein can be introduced simultaneously or sequentially as desired by the user.

### A. Introducing a Cas endonuclease

For purposes of the present disclosure, introduction of a Cas endonuclease can be achieved in any convenient manner, including transfection, transduction, transformation, electroporation, particle bombardment, cell fusion techniques, and the like.

Alternatively, one can employ a Cas endonuclease that has nicking endonuclease activity (*i.e.,* cleaves only one strand of DNA at the target site; also referred to herein as a "Cas nickase") rather than double-strand break activity. Inducing nicks at the targets site does not activate the non-homologous end joining (NHEJ) pathway at the target site as would a double-stranded break, but it may improve homologous recombination between the genomic locus of interest (one that includes or is near to the target site for the Cas nickase) and the donor DNA. Examples of Cas nickases include Cas endonuclease variants as described below.

In certain embodiments, the Cas endonuclease (including, *e.g.,* a Cas nickase) is a Cas9 endonuclease (*see, e.g.,* PCT Publication No. WO2013/141680). Examples of Cas9 endonucleases include those from *Streptococcus sp.* (*e.g., S. pyogenes, S. mutans,* and S. *thermophilus), Campylobacter sp.* (*e.g., C. jejum*)*, Neisseria sp. (e.g., N. meningitides), Francisella sp.* (*e.g., F. novicida*), and *Pasteurella sp.* (*e.g., P. multocida*) (*see, e.g.,* Cas9 endonucleases described in Fonfara *et al.,* 2013). In some embodiments, the Cas endonuclease is encoded by an optimized Cas9 endonuclease gene, *e.g.*, codon optimized for expression in a fungal cell.

In certain instances, the Cas endonuclease gene is operably linked to one or more polynucleotides encoding nuclear localization signals such that the Cas endonuclease/guide polynucleotide complex that is expressed in the cell is efficiently transported to the nucleus. Any convenient nuclear localization signal may be used, *e.g.*, a polynucleotide encoding an SV40 nuclear localization signal present upstream (5') of and in-frame (*i.e.,* operably linked) with the Cas coding region and a polynucleotide encoding a nuclear localization signal derived from the *T. reesei blr2* (blue light regulator 2) gene present downstream (3') and in frame (*i.e.,* operably linked) with the Cas coding region. Other nuclear localization signals can be employed.

In some embodiments, a Cas-expressing microbial cell is obtained by the user, and thus the user does not need to introduce a recombinant DNA construct capable of expressing a Cas endonuclease into the cell, but rather only need introduce a guide polynucleotide into the Cas expressing cell. For example, a fungal cell can first be stably transfected with a Cas expression DNA construct followed by introduction of a guide polynucleotide into the stable Cas expressing cell (either directly or using a guide polynucleotide expressing DNA construct). This set up provides certain advantages as the user can generate a population of stable Cas expressing fungal cells into which different guide polynucleotides can be introduced independently. In other embodiments, more than one guide polynucleotide can be introduced into the same Cas9 expressing cell.

As yet another example, a Cas endonuclease expressing host cell can be used to create a "helper strain" that can provide, in *trans,* the Cas endonuclease to a "target strain". In brief, a heterokaryon can be created between the helper strain and the target strain, *e.g.*, by fusion of protoplasts from each strain or by anastomosis of hyphae depending on the species of filamentous fungus. Maintenance of the heterokaryon will depend on appropriate nutritional and/or other marker genes or mutations in each parental strain and growth on suitable selective medium such that the parental strains are unable to grow, whereas the heterokaryon, due to complementation, is able to grow. Either at the time of heterokaryon formation or subsequently, a guide RNA and a donor DNA are introduced by transfection. The guide RNA may be directly introduced or introduced *via* a DNA construct having a Cas endonuclease expression cassette and a selectable marker gene. The Cas endonuclease is expressed from the gene in the helper strain nucleus and is present in the cytoplasm of the heterokaryon. The Cas endonuclease associates with the guide RNA to create an active complex that is targeted to the desired target site(s) in the genome, where the donor DNA is inserted. Subsequently, spores are recovered from the heterokaryon and subjected to selection or screening to recover the target strain with a donor DNA inserted at the target site. In cases in which an expression cassette is used to introduce the guide RNA, heterokaryons are chosen in which the guide RNA expression construct is not stably maintained.

In some embodiments, a Cas endonuclease is directly transfected in to the microbial cell. In other embodiments, a DNA vector comprising an expression cassette for the Cas endonuclease is transformed into a microbial cell.

### i. A DNA Vector

A DNA construct comprising a nucleic acid encoding a Cas endonuclease can be constructed such that it is suitable to be expressed in a host cell. Because of the known degeneracy in the genetic code, different polynucleotides that encode an identical amino acid sequence can be designed and made with routine skills. It is also known that, depending on the desired host cells, codon optimization may be required prior to attempting expression.

A polynucleotide encoding a Cas endonuclease of the present disclosure can be incorporated into a vector. Vectors can be transferred to a host cell using known transformation techniques, such as those disclosed below.

A suitable vector may be one that can be transformed into and replicated within a host cell. For example, a vector comprising a nucleic acid encoding a Cas endonuclease of the present disclosure can be transformed and replicated in a bacterial host cell as a means of propagating and amplifying the vector. The vector may also be suitably transformed into an expression host, such that the encoding polynucleotide is expressed as a functional Cas endonuclease.

A representative useful vector is pTrex3gM (*see,* U.S. Patent Application Publication No. US 2013/0323798) and pTTT (*see,* U.S. Patent Application Publication No. 2011/0020899), which can be inserted into genome of host. The vectors pTrex3gM and pTTT can both be modified with routine skill such that they comprise and express a polynucleotide encoding a Cas endonuclease of the invention.

A vector useful for this purpose typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the Cas endonuclease to a host cell organelle such as the nucleus. For expression under the direction of control sequences, the nucleic acid sequence of the Cas endonuclease is operably linked to the control sequences in proper manner with respect to expression.

A polynucleotide encoding a Cas endonuclease of the present invention can be operably linked to a promoter, which allows transcription in the host cell. The promoter may be any DNA sequence that shows transcriptional activity in the host cell of choice and may be derived from genes encoding proteins either homologous or heterologous to the host cell, and genes that are inducible or constitutively expressed. Examples of promoters for directing the transcription of the DNA sequence encoding a Cas endonuclease, especially in a bacterial host, include the promoter of the lac operon of *E. coli,* the *Streptomyces coelicolor* agarase gene *dagA* or *celA* promoters, the promoters of the *Bacillus licheniformis* amylase gene (amyL), the promoters of the *Bacillus stearothermophilus* maltogenic amylase gene (amyM), the promoters of the *Bacillus amyloliquefaciens* amylase (amyQ), the promoters of the *Bacillus subtilis xylA* and *xylB* genes, and the like.

For transcription in a fungal host, examples of useful promoters include those derived from the gene encoding *Aspergillus oryzae* TAKA amylase, *Rhizomucor miehei* aspartic proteinase, *Aspergillus niger* neutral α-amylase, *Aspergillus niger* acid stable α-amylase, *Aspergillus niger* glucoamylase, *Rhizomucor miehei* lipase, *Aspergillus oryzae* alkaline protease, *Aspergillus oryzae* triose phosphate isomerase, *Aspergillus nidulans* acetamidase and the like. When a gene encoding a Cas endonuclease is expressed in a bacterial species such as an *E. coli,* a suitable promoter can be selected, for example, from a bacteriophage promoter including a T7 promoter and a phage lambda promoter. Along these lines, examples of suitable promoters for the expression in a yeast species include, but are not limited to, the Gal 1 and Gal 10 promoters of *Saccharomyces cerevisiae* and the *Pichia pastoris* AOX1 or AOX2 promoters. Expression in filamentous fungal host cells often involves *cbh1,* which is an endogenous, inducible promoter from *T. reesei* or constitutive glycolytic promoters (*e.g.,* pki). For example, see Liu *et al.* 2008.

Typically Cas9 is not secreted for the purpose of the present invention. Rather Cas9 is targeted and retained in the nucleus such that the DNA editing occurs within the nucleus. In some embodiments, a Nuclear Localisation signal (NLS) may be added or fused to the Cas9 sequence. In other embodiments, however, for example, in bacteria, such a fusion or addition of NLS sequences is optional.

An expression vector may also comprise a suitable transcription terminator and, in eukaryotes, polyadenylation sequences operably linked to the DNA sequence encoding a Cas endonuclease. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMB1, and pIJ702.

The vector may also comprise a selectable marker, *e.g.*, a gene the product of which complements a defect in the isolated host cell, such as the *dal* genes from *B. subtilis* or *B. licheniformis,* or a gene that confers antibiotic resistance such as, *e.g.,* ampicillin, kanamycin, chloramphenicol or tetracycline resistance. Furthermore, the vector may comprise *Aspergillus* selection markers such as *amdS, argB, niaD* and *xxsC*, a marker giving rise to hygromycin resistance, or the selection may be accomplished by co-transformation, such as known in the art. *See e.g.,* Published PCT Application No. WO 91/17243.

The procedures used to ligate the DNA construct encoding a Cas endonuclease, the promoter, terminator and other elements, respectively, and to insert them into suitable vectors containing the information necessary for replication, are known to persons skilled in the art and readily available. *See, e.g.,* Sambrook *et al.,* 2^{nd} ed., Cold Spring Harbor, 1989, and 3^{rd} ed., 2001.

### ii. Method of Transformation

Introduction of a DNA construct or vector into a host cell includes techniques such as transformation; electroporation; nuclear microinjection; transduction; transfection, *e.g.*, lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g.,* Sambrook *et al.* (2001), *supra.* The expression of heterologous protein in *Trichoderma* is described, for example, in U.S. Patent No. 6,022,725. Reference is also made to Cao *et al.* (2000) for transformation of *Aspergillus* strains. Genetically stable transformants can be constructed with vector systems whereby the nucleic acid encoding a Cas endonuclease is stably integrated into a host cell chromosome. Transformants are then selected and purified by known techniques.

The preparation of *Trichoderma* sp. for transformation, for example, may involve the preparation of protoplasts from fungal mycelia (*e.g., see* Campbell *et al.* 1989). The mycelia can be obtained from germinated vegetative spores. The mycelia are treated with an enzyme that digests the cell wall, resulting in protoplasts. The protoplasts are protected by the presence of an osmotic stabilizer in the suspending medium. These stabilizers include sorbitol, mannitol, potassium chloride, magnesium sulfate, and the like. Usually the concentration of these stabilizers varies between 0.8 M and 1.2 M, *e.g.,* a 1.2 M solution of sorbitol can be used in the suspension medium.

Uptake of DNA into the host *Trichoderma* sp. strain depends upon the calcium ion concentration. Generally, between about 10-50 mM CaCl₂ is used in an uptake solution. Additional suitable compounds include a buffering system, such as TE buffer (10 mM Tris, pH 7.4; 1 mM EDTA) or 10 mM MOPS, pH 6.0 and polyethylene glycol. The polyethylene glycol is believed to fuse the cell membranes, thus permitting the contents of the medium to be delivered into the cytoplasm of the *Trichoderma* sp. strain. This fusion frequently leaves multiple copies of the plasmid DNA integrated into the host chromosome.

Usually transformation of *Trichoderma* sp. uses protoplasts or cells that have been subjected to a permeability treatment, typically at a density of 10⁵ to 10⁷/mL, particularly 2x10⁶/mL. A volume of 100 µL of these protoplasts or cells in an appropriate solution (*e.g.*, 1.2 M sorbitol and 50 mM CaCl₂) may be mixed with the desired DNA. Generally, a high concentration of PEG is added to the uptake solution. From 0.1 to 1 volume of 25% PEG 4000 can be added to the protoplast suspension; however, it is useful to add about 0.25 volumes to the protoplast suspension. Additives, such as dimethyl sulfoxide, heparin, spermidine, potassium chloride and the like, may also be added to the uptake solution to facilitate transformation. Similar procedures are available for other fungal host cells. *See, e.g.,* U.S. Patent No. 6,022,725.

### B. Introducing a guide RNA

In some embodiments, introduction of the guide polynucleotide can be done in any convenient manner, including transfection, transduction, transformation, electroporation, particle bombardment, cell fusion techniques, *etc.*

In certain embodiments, a guide polynucleotide is introduced into the fungal cell by introducing a recombinant DNA construct that includes an expression cassette (or gene) encoding the guide polynucleotide. In some embodiments, the expression cassette is operably linked to a eukaryotic RNA pol III promoter. These promoters are of particular interest as transcription by RNA pol III does not lead to the addition of a 5' cap structure or polyadenylation that occurs upon transcription by RNA polymerase II from an RNA pol II dependent promoter. In certain embodiments, the RNA pol III promoter is a filamentous fungal cell U6 polymerase III promoter.

As another example, a Cas endonuclease expressing host cell can be induced to uptake an *in vitro* synthesized guide RNA to enable Cas endonuclease activity and targeting to a defined site in the genome. In some cases, it will be desirable to induce uptake of both guide RNA and a separate DNA construct bearing a selectable marker gene to allow for selection of those cells that have taken up DNA and, at high frequency, are expected to have simultaneously taken up guide RNA. As above, screening those transformants that show an unstable phenotype with respect to the selectable marker for the genetic modification of interest (*e.g.*, homologous recombination with a donor DNA) without vector DNA insertion is obtained.

For example, a Cas endonuclease expressing host cell can be transformed with a DNA construct including a guide RNA expression cassette containing a second selectable marker (and optionally a separate donor DNA). Host cells that are selected for using the second selectable marker will express the guide RNA from this DNA construct, which enables Cas endonuclease activity and targeting to a defined target site of interest in the genome.

In certain embodiments of the disclosure, the guide polynucleotide is a guide RNA that includes a crRNA region (or crRNA fragment) and/or a tracrRNA region (or tracrRNA fragment) of the type II CRISPR/Cas system that can form a complex with a type II Cas endonuclease. As indicated above, the guide RNA/Cas endonuclease complex can direct the Cas endonuclease to a microbial cell genomic target site, enabling the Cas endonuclease to introduce a double strand break into the genomic target site. In some cases, the RNA that guides the RNA/Cas9 endonuclease complex is a duplex that includes a crRNA and a separate tracrRNA. In other instances, the guide RNA is a single RNA molecule (*e.g.*, a fusion) that includes both a crRNA region and a tracrRNA region (sometimes referred to herein as a fused guide RNA). One advantage of using a fused guide RNA versus a duplexed crRNA-tracrRNA is that only one expression cassette needs to be made to express the fused guide RNA

### C. Introducing a donor DNA

When a double-strand break is induced in the genomic DNA of a host cell (*e.g.,* by the activity of a Cas endonuclease/guide RNA complex at a target site, the complex having double-strand endonuclease activity), the cell's DNA repair mechanism is activated to repair the break, which due to its error-prone nature, can produce mutations at double-strand break sites. The most common repair mechanism to bring the broken ends together is the non-homologous end-joining (NHEJ) pathway. The structural integrity of chromosomes is typically preserved by the repair, however deletions, insertions, or other rearrangements are possible (Siebert and Puchta, 2002; Pacher *et al.,* 2007).

For target specific gene editing, *e.g.*, gene insertion, gene replacement and other sequence integrations, a donor DNA includes a first region and a second region (*i.e.,* homology arms) that are homologous to corresponding first and second regions in the genome of the fungal cell, wherein the regions of homology generally include or surround the target site at which the genomic DNA is cleaved by the Cas endonuclease. These regions of homology promote homologous recombination with their corresponding genomic regions of homology resulting in exchange of DNA between the donor DNA and the genome. As such, the provided methods result in the integration of the polynucleotide of interest of the donor DNA at or near the cleavage site in the target site in the fungal cell genome, thereby altering the original target site, thereby producing an altered genomic target site.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the fungal cell genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or even 100% sequence identity, such that the sequences undergo homologous recombination.

The region of homology on the donor DNA can have homology to any sequence flanking the target site. While in some embodiments the regions of homology share significant sequence homology to the genomic sequence immediately flanking the target site, it is recognized that the regions of homology can be designed to have sufficient homology to regions that may be further 5' or 3' to the target site. In still other embodiments, the regions of homology can also have homology with a fragment of the target site along with downstream genomic regions. In one embodiment, the first region of homology further comprises a first fragment of the target site and the second region of homology comprises a second fragment of the target site, wherein the first and second fragments are dissimilar.

The lengths of the homology arms also contribute to transfection and recombination efficacy and efficiency. It is typically known in the art that such lengths range from 0.5 to 1 kb in order to achieve targeted editing.

It has been surprisingly found in the instant disclosure, that in certain filamentous fungi, homology arms as short as 100 bps or less (*e.g.*, as short as 80 bps or less, as short as 60 bps or less, or even as short as 40 bps or less) in length can be used to achieve efficient homologous recombination stimulated by the guide RNA/Cas endonuclease complex. Moreover, while the traditional double stranded donor DNA (dsDNA) would work to mediate targeted gene editing, as exemplified herein single stranded donor DNA (ssDNA) of the instant disclosure performs equivalently in mediating homologous recombination stimulated by the guide RNA/Cas endonuclease complex, especially when shorter homologous arms are employed (*i.e.,* homology arms as short as 100 bps or less). As such, the multi-step molecular manipulation and targeted gene editing mediated by the Cas system is substantially simplified.

### D. Microbial cells

Microbial cells employed in the methods and compositions disclosed herein may be any fungal host cells from the phyla Ascomycota, Basidiomycota, Chytridiomycota, and Zygomycota (as defined by Hawksworth *et al.,* 1995) as well as the Oomycota (Hawksworth *et al*.,1995) and all mitosporic fungi (Hawksworth *et al.,* 1995). In certain embodiments, the microbial host cells are yeast cells, *e.g., Candida, Hansenula, Kluyveromyces, Pichia, Saccharomyces, Schizosaccharomyces,* or *Yarrowia* cells. Species of yeast include, but are not limited to, *Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, Kluyveromyces lactis,* and *Yarrowia lipolytica.*

In some embodiments, the microbial cells are filamentous fungal cells including, but not limited to, species of *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium, Trichoderma* or *Rasamsonia.* In another preferred example, the filamentous fungal cells are selected from *Aspergillus acufeatus, Aspergillus awamori, Aspergillus fumigatus, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Trichoderma harzianum, Trichoderma koningii, Trichoderma longibrachiatum, Trichoderma reesei, Trichoderma viride, Rasamsonia argillacea, Rasamsonia brevistipitata, Rasamsonia byssochlamydoides, Rasamsonia cylindrospora, Rasamsonia composticola, Rasamsonia eburnean* or *Rasamsonia emersonii.*

In certain embodiments, the microbial host cells are bacterial cells, *e.g.,* a *Bacillus alkalophilus, Bacillus amyloliquefaciens, Bacillus brevis, Bacillus circulans, Bacillus coagulans, Bacillus lautus, Bacillus lentus, Bacillus licheniformis, Bacillus megaterium, Bacillus stearothermophilus, Bacillus subtilis,* or *Bacillus thuringiensis* or a *Streptomyces* such as, *e.g.,* a *Streptomyces lividans* or *Streptomyces murinus* or a gram negative bacterium, such as, *e.g.,* an *E. coli or* a *Pseudomonas sp.*

For the aforementioned species, it is understood that the disclosure and source species would encompass both the perfect and imperfect states of such organisms, and other taxonomic equivalents thereof, *e.g.*, anamorphs, regardless of the species name by which they are known. Those skilled in the art will readily recognize the identity of appropriate equivalents of such source species.

Strains of the above-mentioned species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

### E. The potential target for genome editing

In some embodiments, specific genes are targeted for modification using the disclosed methods, including genes encoding enzymes (*e.g.*, acetyl esterases, aminopeptidases, amylases, arabinases, arabinofuranosidases, carboxypeptidases, catalases, cellulases, chitinases, cutinase, deoxyribonucleases, epimerases, esterases, α-galactosidases, β-galactosidases, α-glucanases, glucan lysases, endo-β-glucanases, glucoamylases, glucose oxidases, α-glucosidases, β-glucosidases, glucuronidases, hemicellulases, hexose oxidases, hydrolases, invertases, isomerases, laccases, lipases, lyases, mannosidases, oxidases, oxidoreductases, pectate lyases, pectin acetyl esterases, pectin depolymerases, pectin methyl esterases, pectinolytic enzymes, peroxidases, phenoloxidases, phytases, polygalacturonases, proteases, rhamno-galacturonases, ribonucleases, transferases, transport proteins, transglutaminases, xylanases, hexose oxidases, and combinations thereof).

There are numerous variations for implementing the methods described herein. For example, instead of having the Cas expression cassette present as an exogenous sequence in the fungal host cell, the Cas expression cassette can be integrated into the genome of the fungal host cell. Generating this parental cell line would allow a user to simply introduce a desired guide RNA (*e.g.*, as a guide RNA expression vector) which would then target the genomic site of interest as detailed elsewhere herein. In some of these embodiments, the integrated Cas gene can be designed to include polynucleotide repeats flanking it for subsequent loop-out/removal from the genome if needed.

### F. Implementing Gene Editing by a Cas endonuclease/guide polynucleotide complex

Virtually any site in a microbial cell genome may be targeted using the disclosed methods and compositions, so long as the target site includes the required protospacer adjacent motif, (hereinafter "PAM"). In the case of the *S. pyogenes* Cas9, the PAM has the sequence NGG (5' to 3'; where N is A, G, C or T), and thus does not impose significant restrictions on the selection of a target site in the genome. Other known Cas9 endonucleases have different PAM sites (*see, e.g.,* Cas9 endonuclease PAM sites described in Fonfara *et al.,* 2013).

The length of at least one of the target sites can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic (*i.e.,* the sequence on one strand reads the same in the opposite direction on the complementary strand). The cleavage site can be within the target sequence or the cleavage site can be outside of the target sequence. In another variation, the cleavage could occur at nucleotide positions immediately opposite each other to produce a blunt end cut or, in other cases, the incisions could be staggered to produce single-stranded overhangs, also called "sticky ends", which can be either 5' overhangs, or 3' overhangs.

In some cases, active variant target sequences in the genome of the fungal cell can also be used, meaning that the target site is not 100% identical to the relevant sequence in the guide polynucleotide (within the crRNA sequence of the guide polynucleotide). Such active variants can comprise at least 65%, 70%, 75%, 80%, 85%, 90%, 91%, 92%, 93%, 94%, 95%, 96%, 97%, 98%, 99% or more sequence identity to the given target site, wherein the active variant target sequences retain biological activity and hence are capable of being recognized and cleaved by a Cas endonuclease. Assays to measure the double-strand break of a target site by an endonuclease are known in the art and generally measure the overall activity and specificity of the agent on DNA substrates containing recognition sites.

Target sites of interest include those located within a region of a gene of interest. Non-limiting examples of regions within a gene of interest include an open reading frame, a promoter, a transcriptional regulatory element, a translational regulatory element, a transcriptional terminator sequence, an mRNA splice site, a protein coding sequence, an intron site, an intron enhancing motif, and the like.

### G. Identifying a microbial cell with desired genome editing

In certain embodiments, modification of the genome of the microbial cell results in a phenotypic effect that can be detected and, in many instances, is a desired outcome of the user. Non-limiting examples include acquisition of a selectable cell growth phenotype (*e.g.*, resistance to or sensitivity to an antibiotic, gain or loss of an auxotrophic characteristic, increased or decreased rate of growth, *etc*.)*,* expression of a detectable marker (*e.g.,* fluorescent marker, cell-surface molecule, chromogenic enzyme, etc.), and the secretion of an enzyme the activity of which can be detected in culture supernatant.

When modification of the genome of the microbial cell results in a phenotypic effect, a donor DNA is often employed that includes a polynucleotide of interest that is (or encodes) a phenotypic marker. Any convenient phenotypic marker can be used, including any selectable or screenable marker that allows one to identify, or select for or against a fungal cell that contains it, often under particular culture conditions. Thus, in some aspects of the present invention, the identification of microbial cells having a desired genome modification includes culturing the microbial population of cells that have received the Cas endonuclease and guide polynucleotide (and optionally a donor DNA) under conditions to select for cells having the modification at the target site. Any type selection system may be employed, including assessing for the gain or loss of an enzymatic activity in the fungal cell (also referred to as a selectable marker), *e.g.*, the acquisition of antibiotic resistance or gain/loss of an auxotrophic marker.

In some instances, the genomic modification in the microbial cells is detected directly using any convenient method, including sequencing, PCR, Southern blot, restriction enzyme analysis, and the like, including combinations of such methods.

The present disclosure is further described by the following examples which should not be construed as limiting the scope of the disclosure.

### EXAMPLES

Aspects of the present methods and compositions may be further understood in light of the following examples, which should not be construed as limiting.

### EXAMPLE 1

### PREPARATION OF DONOR TEMPLATES

### Example 1.1 Preparation of single strand oligonucleotides

Twenty (20) nmole of single strand DNA fragments (ssDNAs) of various lengths: 70-nucleotides, 100-nucleotides and 200-nucleotides, including both upper and lower strands (SEQ ID NOs: 1-6), were produced by Integrated DNA Technologies (IDT) as lyophilized desalted DNA. These 70-nt, 100-nt and 200-nt single strand DNA fragments contained flanking sequences of about 23-24-nucleotides, 40-41-nucleotides, & 90-91-nucleotides, respectively. While the sequences listed below in this example are marked with the designation of "upper strain" or "lower strand", it is believed such a designation is not strictly necessary in that a double-stranded break is made in this example, and either strand can function as a repair template. It is however believed that when Cas nickases are employed, such as described herein, the efficiency of repair would depend on the strand used as repair template.

### Example 1.2 Preparation of double strand oligonucleotides

Double stranded donor DNA templates of various lengths: 330 bps, 730 bps, and 1100 bps, were produced by Integrated DNA Technology (IDT) as 200 ng DNA fragments.

Each of the double strand donor templates contained a 19-nucleotide insertion sequence that was used to replace the entire target site TS2 sequence (SEQ ID NO: 10).

The double strand donor template of 330 bps (SEQ ID NO: 7) has 152 bps of upstream flanking sequence and 159 bps of downstream flanking sequence.

The double strand donor template of 730 bps (SEQ ID NO: 8) has 352 bps of upstream flanking sequence and 359 bps of downstream flanking sequence.

The double strand donor template of 1100 bps (SEQ ID NO: 9) has 562 bps of upstream flanking sequence and 529 bps of downstream flanking sequence.

Insertion sequence between the upstream and downstream flanking sequences contained 3 stop codons in 3 reading frames and the restriction cleavage sites *Pme1 & Pac1.*

### EXAMPLE 2

### TARGET SITE SELECTION AND sgRNA SYNTHESIS

### Example 2.1 Target site selection

The *pyr 4* marker gene was selected to test homologous repair using CRISPR-Cas9 system in the presence of exogenous donor templates including single strand oligonucleotides and double strand DNAs, as described in Example 1 above. The target site with the motif G-N20-GG was selected with the 23-bp sequence of SEQ ID NO: 10, which included the PAM (TGG) site.

The template for sgRNA synthesis was produced by IDT as a DNA fragment with the sequence set forth as SEQ ID NO: 11, which contains the T7 promoter sequence followed by the 20-base target site (in *italic* and underlined text) without the PAM site, guide RNA scaffold, followed by the terminator site (TTTTT).

### Example 2.2 Production of saRNA

Guide RNAs were produced *in vitro* using the MEGA shortscript™ kit (Ambion, Product No. AM 1354). *In vitro* transcription was carried out at 37°C for at least 5 hours. The resulting RNA was purified using Qiagen RNAeasy Plus mini kit (Qiagen). Nano drop was used to determine the amount of RNA produced.

### EXAMPLE 3

### TRANSFORMATION OF GUIDE RNAS AND SINGLE STRAND TEMPLATE

A *Trichoderma reesei* strain was derived from RL-P37 by screening for increased cellulase productivity and having a single point mutation that inactivates the *pyr2* gene making the strain a uridine auxotroph. This strain was transformed with a DNA construct containing an expression cassette for *Streptococcus pyogenes* Cas9 under the control of the pyruvate kinase (pki) promoter and an expression cassette for the *pyr2* gene from *T. reesei* under the control of the its native promoter as described in PCT International Application No. PCT/CN2014/093918. A transformant with the Cas9-pyr2 cassette integrated into the genome and constitutively expressing the Cas9 gene was identified by selecting for cells having a functional pyr2 gene (growth without uridine supplementation on Vogels media).

Protoplasts were prepared from the *T. reesei* strain. The strain was grown on PDA plate for 5 days at 30°C. Spores were collected and inoculated into 50 mL of YEG (5 g/L yeast extract plus 20 g/L glucose) broth in a 250 mL, 4-baffle shake flask, and incubated at 37°C, for 16-20 hours at 200 rpm.

The mycelia were recovered by transferring the liquid volume into 50 mL conical tubes and spinning at 2,500 rpm for 10 minutes. The supernatant was decanted. The mycelial pellet was then transferred into a 250 mL, 0.22 micron CA Corning filter bottle with 40 mL solution containing 2g lysing enzyme (SIGMA). The mixture was thereafter incubated at 30°C, mixing at 200 rpm, for 2 hours to generate protoplasts for transformation.

Protoplasts were harvested by filtration through sterile miracloth into a 50 mL conical tube. They were then pelleted by spinning at 2,000 rpm for 5 minutes and aspirated. The protoplast pellet was washed once with 50 mL of 1.2 M sorbitol, spun down, aspirated, and washed again with 25 mL of sorbitol/CaCl₂.

Protoplasts were counted and then pelleted at 2,000 rpm for 5 minutes, the supernatant was decanted, and the protoplast pellet re-suspended in an amount of sorbitol/CaCl₂ sufficient to generate a protoplast concentration of 1.25×10⁸ protoplasts per mL, generating a protoplast solution.

Aliquots of up to 15 µg of *in vitro* synthesized guide RNA (at 12µg/µL) and 2 µL of 0.1 mM single strand DNA template were placed into 15 mL conical tubes and the tubes were put on ice. Then 200 µL of the protoplast suspension was added along with 50 µL PEG solution to each transformation aliquot. The tubes were mixed gently and incubated on ice for 20 minutes. A PEG solution, in the volume of 4 mL, was added to the transformation aliquot tubes, and these were incubated at room temperature for 5 minutes. Sorbitol/CaCl₂ solution, in the volume of 3.5 mL, was added to the tubes (generating a total volume of 8 ml). The transformation mixture was divided into 2 aliquots each containing about 4 mL. Vogels sorbitol containing 1.0 mg/mL uridine melted top agar (kept molten by holding at 50°C) was mixed with the transformation reaction, which was then plated, and incubated at 30°C for 4-5 days.

Selection was carried out using an overlay agar consisting of Vogels with 1.8 mg/mL FOA (Zymo Research Corp.) and 0.5 mg/mL uridine. Controls were carried out using protoplasts incubated with 2 µL of 0.1 mM single strand DNA template without the guide RNA.

### EXAMPLE 4

### CHARACTERIZATION OF FOA RESISTANT TRANSFORMANTS

Genomic DNA was isolated from *T. reesei* colonies growing on FOA-uridine plates using the hot phenol extraction protocol. Small amount of mycelia (-0.25 cm² mycelia with agar) were transferred to 600 µL eppendorf tubes, grounded and re-suspended in 120 µL Lysis buffer. A lysis buffer was prepared by blending 200 µL of 1M Tris, at pH8, 200 µL of 3M sodium acetate pH5.8, and 200 µL of phenol:chloroform:isoamyl alcohol blend (25:24:1, v/v), and 1,800 µL of a TE buffer prepared with 10 mM Tris-HCI, pH 8, and 0.1 mM EDTA. Chloroform (120 µL) was subsequently added to the lysed mycelia, mixed by vortexing and incubated in a thermomixer for 6 minutes at 72°C. The lysate was then mixed briefly and centrifuged for 3 minutes at maximum speed. The aqueous phase was transferred to a tube containing 100 µL of isopropanol, mixed and centrifuged for 10 minutes. The pellet was washed with 1 mL 70% ethanol and centrifuged. The pellet was re-suspended in 60 µL TE buffer, incubated at 72°C and used as template for PCR.

PCR reactions were carried out in 25 µL reaction volume using 1 µL of genomic DNA, 0.1 µL each of 50 µM primers (forward & reverse primer), 0.25 µL of PCR nucleotide mix and 0.25 µL PfuUltra II DNA polymerase (Agilent Technologies).

The following primers were used to PCR across the *pyr4* target site TS2. Primer pairs with:
SEQ ID NO: 12 - 1F: 5'-CCATCTTGGCTGACGAAAAAGGTCTG-3'; and
SEQ ID NO: 13 - 1R: 5'-CATGCAAAGATACACATCAATCGCAGCTG-3'.

These primers were used when using the single strand DNA as donor template. Primer pairs:
SEQ ID NO: 14 - MH179 5'-CATCGACTACTGCTGCTCTGCTC-3'; and
SEQ ID NO: 15 - MH1805'ATCGCAGCTGGGGTACAATCATC-3'
were used to PCR colonies from transformations using the double strand DNA as donor templates. PCR products were analyzed by electrophoresis using 0.8% agarose gels. PCR products were purified using Qiaquick PCR Purification kit (Qiagen; Catalogue No. 28104).

DNA sequencing was carried out by Sequetech Corp (Moutainview, CA) using sequencing primers pMH094 (SEQ ID NO:16)

Restriction digestions were carried out for 15 minutes at 37°C in 20 µL volume using 5 µL of PCR product and restriction enzymes from New England Biolabs (Pac1, Catalog No. R5547S, Pme1, Catalogue No. R0560S). Reaction products were analyzed by electrophoresis on a 1% agarose gel.

### EXAMPLE 5

### CAS9-MEDIATED HOMOLOGY DIRECTED REPAIR USING SINGLE STRAND OLIGONUCLEOTIDES OF 100 NUCLEOTIDES AS DONOR DNA TEMPLATE

The donor DNA template (100-nucleotides) included two single strand DNAs: (1) SEQ ID NO:4, which is the lower strand (and is the strand complementary to target site TS2 of SEQ ID NO:10); and (2) SEQ ID NO: 3, which is the upper strand and is the same strand as TS2.

SEQ ID NO: 3 and 4 each included a 19-nucleotide stop codon sequence flanked by a 40-nucleotide (5') homology arm and 41-nucleotide (3') homology arm (as shown in FIG. 1).

A single strand DNA of 70 bases (SEQ ID NOs: 1 & 2) was also designed with homology arms of 23-24 nucleotides flanking the 19-nucleotide insertion sequence, and 200 bases (SEQ ID NOs: 5 & 6) with homology arms of 90-91 nucleotides flanking the 19-nucleotide insertion sequence.

In 5 out of 10 FOA resistant strains that were isolated, a 1,200-bp PCR product containing the restriction sites *Pme1* & *Pac1* was obtained and sequenced. The efficient disruption and repair was observed in 50% of the FOA resistant colonies using single strand oligonucleotides of 100 bases with a 40-nucleotide 5' homology arm and 41-nucleotide 3' homology arm flanking the stop codon insertion at the *pyr4* gene locus (shown in FIG. 2).

DNA sequence alignments are depicted in FIG. 4A, using single strand DNA as donor template for repairing by homologous recombination (HR). The PCR products amplified from FOA resistant strains were purified and sequenced using SEQ ID NO:16. Sequence analysis of individual repair events revealed that the *pyr4* gene contains the single strand DNA repair template in 5 out of 10 clones.

The remaining 5 clones contained indels, indicating site-specific induction of DSBs and repairing *via* the non-homologous end joining pathway (NHEJ). The sequencing result was consistent with restriction digestion results in FIG. 2.

PCR products digested by *Pac1* & *Pme1* indicated that homologous recombination *via* the homology directed repair (HDR) pathway has occurred and led to the presence of the 19-nucleotide insertion sequence with 2 restriction sites.

### EXAMPLE 6

### CAS9-MEDIATED HOMOLOGY DIRECTED REPAIR USING SINGLE STRAND OLIGONUCLEOTIDES OF 200 BASES AS DONOR DNA TEMPLATE

Mycelia from 10 FOA resistant colonies were isolated and genomic DNA extractions were carried out. The results are shown in FIG. 3.

The sequence of the 200 bases single strand DNA (SEQ ID NO: 6) was used in transformation experiments. Agarose gel electrophoresis (0.8% agarose gel) was used to analyze the PCR product (1.2 kb band) derived using 1F & 1R primers (SEQ ID NO: 12 & 13). Restriction digestion using *Pac1* revealed the presence of the *Pac1* site in 4 out of 10 PCR products. A frequency of homology directed repair was observed in 40% of the strains (as shown in FIG. 3).

All 10 PCR products were purified and subjected to DNA sequencing using sequencing primer SEQ ID NO: 16. FIG. 4B presents the results of such sequencing and analyses of individual repair events, which revealed that the *pyr4* gene contained the single strand DNA repair template in 4 out of 10 clones.

The remaining 6 clones contained indels, indicating Cas9 induced DSBs and executed repair using the non-homologous end joining pathway (NHEJ).

The sequencing result was consistent with restriction digestion results as shown in Figure 3. These results confirmed that the stimulation of homology-directed repairs (HDR) in the presence of the 200-nucleotide single strand DNA donor template in addition to the NHEJ repair pathways, induced by the Cas9 mediated double strand breaks. In conclusion, homology flanking sequences of 90-91 bases are able to stimulate repair by HDR in fungi.

### EXAMPLE 7

### CAS9 MEDIATED HOMOLOGY DIRECTED REPAIR USING DOUBLE STRAND DNA AS DONOR DNA TEMPLATE

Double strand DNA templates have traditionally been used in gene replacement experiments with 500 bps as the minimum length of flanking homology sequences. The goal of this example is to test whether the double strand DNA template with the shorter flanking homology sequences from 150 bps to up to 500 bps (SEQ ID NOs: 7, 8 and 9) can also induce homologous recombination.

FIG. 5 depicts the double strand DNA templates used, each comprising insertion codons and the almost symmetrical flanking homologous arms.

Agarose gel electrophoresis of PCR products indicated the presence of an unusually higher molecular weight bands faintly visible in Gel B of FIG. 6, lanes 2 and 4. This might have reflected aberrant repair events, possibly caused by concatamerization of the donor template or non-specific recombination products.

In Gel C, restriction enzyme digestion of the PCR products indicated the presence of the *Pac1* site within the *pyr4* gene. This was observed in 4 out of 10 products, indicating that 40% of the experiments/colonies had Cas9 mediated homology directed repair and 60% of the experiments/colonies had NHEJ-related repair.

Gene replacement experiments were conducted using a 1,100-bp double strand donor template with symmetrical homology arms of about 500 bps. The donor DNA also contained a stop insertion with restriction sites, which was used for diagnosing and confirming FOA resistant colonies.

Agarose gel analysis was performed on each of the PCR products amplified from genomic DNA of individual FOA resistant colonies.

As indicated in FIG. 6, agarose gel B, lanes 4 and 8 indicated PCR products with low molecular weights as compared to those products from the control sample (C), indicating large deletions in the *pyr4* gene. Restriction digestions with *Pad1* demonstrated that, aside from sample #5 (as shown in Agarose gel C lane 5), a majority of the PCR products were not digested or digestable with *Pac1.* This indicated that HDR occurred at a low frequency whereas the NHEJ repair pathway occurred predominantly.

### EXAMPLE 8

### HOMOLOGY DIRECTED REPAIR USING CRISPR-CAS9 & DONOR DNA TEMPLATES (ssODN) IN ASPERGILLUS

An *Aspergillus tubigensis* overproducing strain 3M-43/pyrA (*see,* Nikolaev *et al.,* 2013) was used to conduct genomic editing, with the goal of disrupting the L-arabitol dehydrogenase (LDA) pathway, using Cas9. As described in Nikolaev *et al.* (2013), disruption of the L-arabitol dehydrogenase gene pathway was expected to lead to an increased xylanase production by that strain when cultivated under suitable conditions.

Nikolaev *et al.* (2013) further reports that increased xylanase production from the LDA-disrupted *Aspergillus tubigensis* strain 3M-43 is mediated by the transcription factor xlnR; and that a single resulting strain out of 80 attempted contained a disrupted *xlnR* gene using the conventional gene replacement strategy, indicating that the conventional gene replacement strategy (while workable) was inefficient and lacked robustness at best.

In the present example, ultramers of 100-200 bases in both upper and lower strands can be purchased from Integrated DNA Technologies (IDT). Four (4) different single strand oligonucleotides having SEQ ID NOs: 17-20 can also be made by IDT, to be used as donor templates for homology directed repair of the Cas9 induced double strand breaks.

More particularly, SEQ ID NO:17 is a 100-base ultramer upper strand with the 19-base stop codon insertion (in uppercase), with homology arms of 44 bases at the 5' end and 37 bases at the 3'end:

SEQ ID NO: 18 is a 100-base ultramer lower strand with the 19-base stop codon insertion (in uppercase) with homology arms of 37 bases at the 5' end and 44 bases at the 3'end:

SEQ ID NO:19 is a 200-ultramer upper strand with the 19-base stop codon insertion (in uppercase) with homology arms of 94 bases at the 5' end and 87 bases at the 3' end:

SEQ ID NO:20 is a 200-ultramer lower strand with the 19-base stop codon insertion (in uppercase) with homology arms of 87 bases at the 5' end and 94 bases at the 3' end:

As presented above, the oligonucleotides that are 100 bases long can contain a 19 bases long stop codon flanked by 5' and 3' homology arms of 44 and 37 bases (SEQ ID NOs: 17 and 18), respectively.

The oligonucleotides that are 200 bases long can also contain the 19-base stop codon insertion flanked by homology arms of 94 and 87 bases at the 5' and 3', respectively (SEQ ID NOs: 19 and 20).

A Cas9 expression vector pGdpA:Cas9 can be constructed using the codon optimized Cas9 gene (*i.e.,* codon optimized for *Trichoderma reesei expression*), as provided herein). The *Aspergillus nidulans* glyceraldehyde-3-phosphate dehydrogenase gene (*gpdA*) promoter, the 5' untranslated region of *gpdA* mRNA, and the *Aspergillus nidulans trp* C terminator can be used to drive the expression of the Cas9 encoding sequence. The 3.9 kg Xba1 fragment of the *Aspergillus niger pyr*A gene can be inserted into the pGpd:Cas9 plasmid, to be used as a selection marker.

Fungal co-transformation can be carried out using 2 µg of the Cas9 expression vector thus constructed, with *pyr*A selection, 20 µg of *in vitro* synthesized guide RNA and 100 µM of either a 100- or a 200-base single strand ultramer, containing the stop codon of SEQ ID NO:21 in three reading frames: SEQ ID NO: 21(CGTTTAAACCTTAATTAAG)

The *xlnR* gene encodes a zinc binuclear cluster Zn2Cys6 protein. A 20-bp target sequence (SEQ ID NO:22) can be chosen: SEQ ID NO:22: (CAACTCCGAACGAAA TGCGA).

SEQ ID NO:22 precedes the PAM site "CGG" as the target site for Cas9 induced double strand break, as it is located within the zinc binuclear DNA binding domain near the N-terminus of *xlnR.*

A template sequence for *in vitro* synthesis of the guide RNA containing the T7 promoter (underlined), the 20-bp target site (uppercase), the tracr sequence (SEQ ID NO:23) can be ordered as gblocks from IDT and the guide RNAs can then be synthesized *in vitro* using the Megashort Script Kit (Ambion).

SEQ ID NO: 23 is a template for gRNA synthesis *in vitro:*

Co-transformation can be carried out by preparing a transformation mixture with protoplasts from *Aspergillus tugingensis* 3M-43/pyrA strain and the Cas9 expression vector with *pryA* selection as described above, at an amount of 2 µg, the *in vitro* synthesized guide RNA, at the amount of 20 µg, and a 100-base or 200-base single strand ultramer donor template. The transformation mixture thus prepared is then plated onto minimal media plates containing per liter, 6 g NaNO₃, 1.5 g KH₂PO₄, 0.5 g MgSO₄·7H₂O, 0.5 g KCI, Vishniac trace elements, 1.5% agar, and 20 g fructose as a carbon source (pH 6.0).

The colonies that appear on the minimal agar plates following inducation are then transferred to a new minimal agar plate containing D-xylose and xylan as carbon sources.

Several transformants will demonstrate reduced growth on D-xylose or even complete absence of xylanase activity, as can be assayed or estimated based on the halo formation on xylan-based agar plates. The reduced growth or complete absence of xylanase activity are good indicators that the disruption of transcription factor is successful.

Colonies can also be screened for endoxylanase (EXL) activity after growth in liquid culture on 3% sugar beet pulp (SBP) substrate and wheat bran (WB) for 5 days at 34°C. Mutation(s) in the *xlnR* gene can be confirmed by the absence of xylanase activity. Such a deletion or mutation can also be confirmed with PCR using genomic DNA and *xlnR* gene specific primers of SEQ ID NO: 24 (forward primer) and SEQ ID NO: 25 (reverse primer), in colonies manifesting the *xlnR* gene knockout phenotype.PCR products of about 3,820 kb in size can be generated in all transformants. Those transformants with reduced growth on D-xylose and absence of xylanase activity will show PCR products containing the *pme1* and *pac1* sites. Restriction digestion can be used to confirm the presence of the sites, resulting in 2 bands of about 1,100 bp and 2,700 bp, in size. These bands would confirm the successful Cas9-induced double strand breaks and homology directed repair using single strand oligonucleotides acting as donor repair templates in *Aspergillus tubigensis.*

The results will also demonstrate that using the CRISPR-Cas9 strategy is a much more efficient means to achieve gene editing in a filamentous fungal strain such as an *Aspergillus,* as compared to the conventional means of modifying or disrupting genes in fungal strains.

### EXAMPLE 9

### EXPRESSION OF CAS9 IN BACILLUS SUBTILIS

### 1. Construction of pSB-SpyCas9 Expression Vector Set Forth in FIG. 8

The Spel-HindIII fragment (4.2 kb) carrying the SpyCas9 gene (SEQ ID NO: 26) was ligated into pSB cut with the same enzymes (resulting a fragment of 5.6 kb). More particularly, the polynucleotide of SEQ ID NO:26 is a sequence of Cas9 of *Streptococcus pyogenes* M1 GAS (Locus Spy_1046), with the Ndel-Xhol fragment, and the BsrDI restriction site marked by **bold** texts. The C-terminal underlined texts mark the nuclear localization sequence and deca-His tag.

The ligation mix was then used to transform *Bacillus subtilis* C2987 cells and about 100 transformants were obtained. Eight (8) colonies were picked, and their sequences were confirmed after mini-prep. Those were then pooled and used to transform CB20-1 and *Bacillus subtilis* 168 cells.

Two transformants of Spy-Cas9 (A and B) were picked and grown in individual 2-mL pre-cultures made with LB and 10 ppm neomycin. A volume of 1 mL of the pre-culture was used to inoculate 35 mL of Grant's II Medium with 10 ppm neomycin. The cultures were then grown for about 63 hours at 37°C, shaking at 280 rpm, and maintained at 70% humidity in Ultra-Yield Flasks using enhanced seals.

After the cultures are completed, the broths were centrifuged and the cell pellets and supernatants were stored separately. A cell pellet was taken from 1 mL of cells out of each of the cultures, and the pellets were suspended in 0.5 mL of Buffer P1. Five (5) mL of Ready-Lyse (a T4 lysozyme) was then added to each mixture. The mixtures were incubated at 37°C for about 0.5 hour.

The cultures were observed to become viscous at the end of 0.5 hour. An Omnicleave nuclease at the volume of 5 mL was added and the incubation was carried on for another 0.5 hour. While the samples were still turbid, the mixture has reduced viscosity as a result of lysis. The lysed cell pellets were then put onto SDS-PAGE and His-Tag detection was carried out using Western Blots. Expression of SpyCas9 was observed.

### 2. Targeting and editing the phrA gene of Bacillus subtilis

The sequence of the *phrA* gene, which is involved in the early sporulation pathway of *Bacillus subtilis,* is presented below as SEQ ID NO: 27, with the targeting site underlined.

Deletion of the *phrA* gene was expected to cause uncontrolled RapA phosphatase activity with the consequence of non-initiation of sporulation (*see,* Proc. Natl. Acad. Sci. USA, 94 (1997): 8612-8616).

The targeting sequence, underlined above and presented herein as SEQ ID NO: 28, included the PAM site "AGG", was used as the target site for Cas9 activity. A T7 promoter was added preceding the 20-bp *phrA* sequence without the PAM site nucleotides, and the guide scaffold sequence was added to the 3' end. The resulting sequence was used as a template for *in vitro* guide RNA synthesis, applying the MegaShort Script Kit (Ambion) and the RNAEasy kit (Qiagen), which was used for guide RNA purification.

A wild type *Bacillus subtilis* strain 168 (trpC2) was obtained from the *Bacillus* Genetic Stock Center. A transformation mixture comprising the Cas9 expression plasmid with 2 ultramers (154-base single stranded upper and lower strand oligonucleotides containing the entire *phrA* open reading frame having a 19-base stop codon insertion), and the *in vitro* synthesized guide RNA as described above, was then grown for about 30 hours at 37°C in Schaeffer's sporulation medium.

The control strains without the Cas9 expression plasmid were compared with the strains expressing Cas9. The percentage of sporulation of Cas9 and non-Cas9 was calculated and presented as ratios of spore counts versus viable cell counts. It was observed that sporulation was abolished in the Cas9 strains, which indicated the disruption of the *phrA* gene.

The primer pairs of SEQ ID NO: 29 (forward primer) and SEQ ID NO: 30 (reverse primer) were used for colony PCR.

PCR amplification of the *phrA* gene and subsequent restriction digestion using *Pmel* or *Pacl* showed a double band of about 70-80 bases on a 4% agarose gel. This indicated that homologous directed repair of the *phrA* gene using the donor signal strand oligonucleotides was achieved.

### EXAMPLE 10

### EXPRESSION OF STREPTOCOCCUS PYOGENES CAS9 IN STREPTOMYCES LIVIDANS

In the literature Cas9-mediated, targeted chromosomal deletions were reported in three different types of *Streptomyces* strains with varied efficiencies ranging from 70 to 100% (*See,* ACS Synthetic Biology (2015) 19:4(6) 723-728). This reported work used donor templates of several kilobases in length. The gene encoding *S. pyogenes* Cas9, codon optimized for expression in *Streptomyces lividans* is set forth in SEQ ID NO: 31.

In the present experiment, donor templates including ultramers in the form of single strand oligonucleotides were designed to effect Cas9-mediated gene editing in *Streptomyces.* Specifically, CRISPR-Cas9 can be used to delete two *Streptomyces* genes, *sco7700* and *sco7701,* which belong to a two-gene operon responsible for methylisoborneol (MIB) biosynthesis. Methylisoborneol is a volatile organic compound produced by *Streptomyces,* which is thought to be responsible for the characteristic smell of moist soil as well as a number of unpleasant tastes or odors that is often deemed undesirable or even problematic in large scale fermentation plants. *See,* J. Am. Chem. Soc. (2008) 16: 130(28): 8908-8909.

FIG. 10 depicts the expression cassette with the Cas9 gene and the guide RNA sequence together with the 20 bp target site of SEQ ID NO: 32, with the 2 kb homology repair donor in a plasmid as control. FIG. 11 depicts the expression cassette without the 2 kb homology repair donor template, in order to allow for the use of 200-base ultramers with stop codons.

Two (2) µg of the Cas9-gRNA expression cassette and 100 µM of ultramers of 200 bases oligonucleotides (SEQ ID NO: 33) was used to disrupt the *sco7700* and *sco7701* genes.

Disruption of the MIB genes was confirmed using the absence of odor from a 50 mL culture cultivated at 30°C. PCR amplification of the MIB gene, followed by *Pmel* or *Pacl* restriction digestion further, more precisely, verified the disruption of the MIB gene.

### REFERENCES

PCT Publication No. WO 1991/17243
PCT Publication No. WO 2013/141680
PCT International Application No. PCT/CN2014/093918
U.S. Patent Application Publication No. 2011/0020899
U.S. Patent Application Publication No. 2013/0323798
U.S. Patent No. 5,107,065
U.S. Patent No. 6,022,725
U.S. Patent No. 8,697,359
Bleuyard et al., DNA Repair 5:1-12, 2006*.*
ACS Synthetic Biology (2015) 19:4(6) 723-8
J. Am. Chem. Soc. (2008) 16: 130(28):8908-9.
Sambrook et al., MOLECULAR CLONING: A LABORATORY MANUAL, 2nd ed., Cold Spring Harbor, 1989, and 3rd ed., 2001.
Cong et al., Science, 339:819-823, 2013*.*
Siebert and Puchta, Plant Cell 14:1121-1131, 2002*.*
Fonfara et al., Nucleic Acids Res., pages 1-14, 2013*.*
Pacher et al., Genetics 175:21-29, 2007*.*
Cao et al., Science 9:991-1001, 2000*.*
Campbell et al., Curr. Genet. 16:53-56, 1989*.*
Fonfara et al., Nucleic Acids Res., pages 1-14, 2013*.*
Hawksworth et al., "Ainsworth and Bisby's Dictionary of The Fungi", 8th edition, 1995, CAB International, University Press, Cambridge, UK*.*
Henikoff and Henikoff, Proc. Natl. Acad. Sci. USA 89:10915, 1989*.*
Higgins and Sharp, CABIOS 5:151-153, 1989*.*
Higgins et al., Comput. Appl. Biosci. 8:189-191, 1992*.*
Hsu et al., "Development and Applications of CRISPR-Cas9 for Genome Engineering", Cell 157: 1262-1278, 2014*.*
Liu et al., "Efficient genome editing in filamentous fungus Trichoderma reesei using the CRISPR/Cas9 system", Cell Discovery, Vol. 1 (No. 15007), 2015*.*
Liu et al., Acta Biochim. Biophys. Sin (Shanghai) 40(2): 158-165, 2008*.*
Needleman and Wunsch, J. Mol. Biol. 48:443-453, 1970*.*
Nikolaev et al., Biotechnol. J. (8):905-911, 2013*.*
Rudin et al., "Genetic and Physical Analysis of Double-Strand Break Repair and Recombination in Saccharomyces Cerevisiae", Genetics 122:519-534, 1989*.*
Smith et al., Nucl. Acids Res. 23:5012-5019.
Matsu-Ura et al. "Efficient gene editing in Neurospora crassa with CRISPR technology." Fungal biology and biotechnology 2:4, 2015.
Nødvig et al. "A CRISPR-Cas9 System for Genetic Engineering of Filamentous Fungi." PLoS One 10(7):e0133085, 2015
Arazoe et al. "Tailor-made CRISPR/Cas system for highly efficient targeted gene replacement in the rice blast fungus." Biotechnology and bioengineering 112(12): 2543-9, 2015
Arazoe et al. "Site-specific DNA double-strand break generated by I-Scel endonuclease enhances ectopic homologous recombination in Pyricularia oryzae." FEMS microbiology letters 352(2): 221-9, 2014
DiCarlo et al. "Genome engineering in Saccharomyces cerevisiae using CRISPR-Cas systems." Nucleic acids research 41 (7): 4336-43, 2013

## Claims

1. A method for genome editing in a filamentous fungal cell, the method comprising introducing into the filamentous fungal cell a Cas endonuclease, a guide polynucleotide, and a donor polynucleotide, wherein the donor polynucleotide comprises at least one homology arm, wherein the homology arm is less than 150 nucleotides in length and comprises sequence homology to a targeted genomic locus of the fungal cell, wherein the Cas endonuclease and guide polynucleotide form a complex that enables the Cas endonuclease to act at or near the targeted genomic locus of the fungal cell, and wherein the donor polynucleotide is a single strand DNA.

2. The method of claim 1, wherein the donor polynucleotide is inserted into the targeted genomic locus of the fungal cell, optionally wherein the inserted donor polynucleotide comprises a genome modification selected from the group consisting of a DNA deletion, a DNA disruption, a DNA insertion, a DNA inversion, a DNA point mutation, a DNA replacement, a DNA knock-in, a DNA knock-out and a DNA knock-down.

3. The method of claim 1, wherein the donor polynucleotide further comprises a nucleotide sequence of interest which is either upstream (5') and operably linked to the homology arm or downstream (3') and operably linked to the homology arm.

4. The method of claim 3, wherein the nucleotide sequence of interest is inserted into the targeted genomic locus of the fungal cell, optionally wherein the inserted nucleotide sequence of interest comprises a genome modification selected from the group consisting of a DNA deletion, a DNA disruption, a DNA insertion, a DNA inversion, a DNA point mutation, a DNA replacement, a DNA knock-in, a DNA knock-out and a DNA knock-down.

5. The method of claim 1, wherein the homology arm is between 100-40 nucleotides in length.

6. The method of claim 1, wherein the donor polynucleotide comprises a homology arm upstream (5') and operably linked to a nucleotide sequence of interest and a homology arm downstream (3') and operably linked to the same nucleotide sequence of interest, wherein at least one of the two homology arms are less than 150 nucleotides in length.

7. The method of claim 6, wherein the nucleotide sequence of interest is inserted into the targeted genomic locus of the fungal cell.

8. The method of claim 6, wherein at least one homology arm is between 100-40 nucleotides in length

9. The method of claim 6, wherein both homology arms are less than 500 nucleotides.

10. The method of claim 3 or claim 6, wherein the nucleotide sequence of interest comprises at least one heterologous nucleotide, or wherein the nucleotide sequence of interest comprises a heterologous polynucleotide sequence.

11. The method of claim 1, wherein the Cas endonuclease is a Cas nickase or a functional variant thereof.

12. The method of claim 1, wherein the Cas endonuclease is a Cas9 endonuclease or a functional variant thereof, optionally wherein the Cas9 endonuclease is a species selected from the group consisting of *Streptococcus sp., Campylobacter sp., Neisseria sp., Francisella sp.* and *Pasteurella sp.*

13. The method of claim 1, wherein
(i) the introducing step comprises introducing a polynucleotide construct comprising an expression cassette for expressing the Cas endonuclease or a functional variant thereof in the fungal cell;
(ii) the introducing step comprises introducing a polynucleotide construct comprising an expression cassette for expressing the guide RNA in the fungal cell;
(iii) the introducing step comprises introducing into the fungal cell a circular polynucleotide construct comprising an expression cassette for the Cas endonuclease, an expression cassette for the guide RNA, and the donor DNA; or
(iv) the introducing step comprises directly introducing the guide polynucleotide or Cas endonuclease into the fungal cell.

14. The method of claim 1, wherein the Cas endonuclease or a functional variant thereof is operably linked to a nuclear localization signal.

15. The method of claim 1, wherein the filamentous fungal cell is selected from the genus consisting of *Trichoderma, Penicillium, Aspergillus, Humicola, Chrysosporium, Fusarium, Myceliophthora, Neurospora* and *Emericella.*

## Patentansprüche

1. Verfahren zum Genom-Editieren in einer Fadenpilzzelle, wobei das Verfahren das Einführen einer Cas-Endonuclease, eines Guide-Polynucleotids und eines Donor-Polynucleotids in die Fadenpilzzelle umfasst, wobei das Donor-Polynucleotid mindestens einen Homologie-Arm umfasst, wobei der Homologie-Arm eine Länge von weniger als 150 Nucleotide hat und Sequenzhomologie zu einer genomischen Target-Stelle der Pilzzelle umfasst, wobei die Cas-Endonuclease und das Guide-Polynucleotid einen Komplex bilden, der es der Cas-Endonuclease ermöglicht, am oder in der Nähe der genomischen Target-Stelle der Pilzzelle zu wirken, und wobei das Donor-Polynucleotid eine einzelsträngige DNA ist.

2. Verfahren nach Anspruch 1, wobei das Donor-Polynucleotid in die genomische Target-Stelle der Pilzzelle insertiert wird, wahlweise wobei das insertierte Donor-Polynucleotid eine Genom-Modifikation umfasst, die ausgewählt ist aus der Gruppe bestehend aus einer DNA-Deletion, einer DNA-Disruption, einer DNA-Insertion, einer DNA-Inversion, einer DNA-Punktmutation, einem DNA-Austausch, einem DNA-Knock-in, einem DNA-Knock-out und einem DNA-Knock-down.

3. Verfahren nach Anspruch 1, wobei das Donor-Polynucleotid ferner eine Nucleotidsequenz umfasst, die von Interesse ist, die sich entweder stromaufwärts (5') befindet und funktionsfähig mit dem Homologie-Arm verknüpft ist, oder sich stromabwärts (3') befindet und funktionsfähig mit dem Homologie-Arm verknüpft ist.

4. Verfahren nach Anspruch 3, wobei die Nucleotidsequenz, die von Interesse ist, in die genomische Zielstelle der Pilzzelle insertiert wird, wahlweise wobei die insertierte Nucleotidsequenz, die von Interesse ist, eine Genom-Modifikation umfasst, die ausgewählt ist aus der Gruppe bestehend aus einer DNA-Deletion, einer DNA-Disruption, einer DNA-Insertion, einer DNA-Inversion, einer DNA-Punktmutation, einem DNA-Austausch, einem DNA-Knock-in, einem DNA-Knock-out und einem DNA-Knock-down.

5. Verfahren nach Anspruch 1, wobei der Homologiearm eine Länge zwischen 100 und 40 Nucleotiden hat.

6. Verfahren nach Anspruch 1, wobei das Donor-Polynucleotid einen Homologiearm stromaufwärts (5') und funktionsfähig verknüpft mit einer Nucleotidsequenz, die von Interesse ist, und einen Homologiearm stromabwärts (3') und funktionsfähig verknüpft mit der gleichen Nucleotidsequenz, die von Interesse ist, umfasst, wobei mindestens einer der zwei Homologiearme eine Länge von weniger als 150 Nucleotide hat.

7. Verfahren nach Anspruch 6, wobei die Nucleotidsequenz, die von Interesse ist, in die genomische Target-Stelle der Pilzzelle insertiert wird.

8. Verfahren nach Anspruch 6, wobei mindestens ein Homologiearm eine Länge zwischen 100 und 40 Nucleotide hat.

9. Verfahren nach Anspruch 6, wobei beide Homologiearme kleiner sind als 500 Nucleotide.

10. Verfahren nach Anspruch 3 oder Anspruch 6, wobei die Nucleotidsequenz, die von Interesse ist, mindestens ein heterologes Nucleotid umfasst, oder wobei die Nucleotidsequenz, die von Interesse ist, eine heterologe Polynucleotidsequenz umfasst.

11. Verfahren nach Anspruch 1, wobei die Cas-Endonuclease eine Cas-Nickase oder eine funktionelle Variante davon ist.

12. Verfahren nach Anspruch 1, wobei die Cas-Endonuclease eine Cas9-Endonuclease oder eine funktionelle Variante davon ist, wahlweise wobei die Cas9-Endonuclease eine Spezies ist, die ausgewählt ist aus der Gruppe bestehend aus *Streptococcus sp., Campylobacter sp., Neisseria sp., Francisella sp. und Pasteurella sp.*

13. Verfahren nach Anspruch 1, wobei:
(i) der Schritt des Einführens Einführen eines Polynucleotid-Konstrukts umfasst, umfassend eine Expressionskassette zum Exprimieren der Cas-Endonuclease oder einer funktionellen Variante davon, in die Pilzzelle umfasst;
(ii) der Schritt des Einführens Einführen eines Polynucleotid-Konstrukts umfasst, umfassend eine Expressionskassette zum Exprimieren der Guide-RNA in die Pilzzelle umfasst;
(iii) der Schritt des Einführens Einführen eines zirkularen Polynucleotid-Konstrukts in die Pilzzelle umfasst, umfassend eine Expressionskassette für die Cas-Endonuclease, eine Expressionskassette für die Guide-RNA und die Donor-DNA; oder
(iv) der Schritt des Einführens direktes Einführen des Guide-Polynucleotids oder der Cas-Endonuclease in die Pilzzelle umfasst.

14. Verfahren nach Anspruch 1, wobei die Cas-Endonuclease oder eine funktionelle Variante davon funktionsfähig mit einem nukleären Lokalisationssignal verknüpft ist.

15. Verfahren nach Anspruch 1, wobei die Fadenpilzzelle ausgewählt ist aus der Gattung bestehend aus *Trichoderma, Penicillium, Aspergillus, Humicola, Chrysosporium, Fusarium, Myceliophthora, Neurospora, Hypocrea* und *Emericella.*

## Revendications

1. Procédé pour l'édition d'un génome dans une cellule de champignon filamenteux, le procédé comprenant l'introduction dans la cellule de champignon filamenteux d'une Cas endonucléase, d'un polynucléotide de guidage et d'un polynucléotide donneur, dans lequel le polynucléotide donneur comprend au moins un bras d'homologie, dans lequel le bras d'homologie est de moins de 150 nucléotides de longueur et comprend une homologie de séquence avec un locus génomique ciblé de la cellule fongique, dans lequel la Cas endonucléase et le polynucléotide de guidage forment un complexe qui permet à la Cas endonucléase d'agir au niveau de ou près du locus génomique ciblé de la cellule fongique et dans lequel le polynucléotide donneur est un ADN simple brin.

2. Procédé selon la revendication 1, dans lequel le polynucléotide donneur est inséré dans le locus génomique ciblé de la cellule fongique, optionnellement dans lequel le polynucléotide donneur inséré comprend une modification de génome choisie dans le groupe constitué de: une délétion d'ADN, une disruption d'ADN, une insertion d'ADN, une inversion d'ADN, une mutation ponctuelle d'ADN, un remplacement d'ADN, une activation d'ADN, une inactivation d'ADN et un affaiblissement d'ADN.

3. Procédé selon la revendication 1, dans lequel le polynucléotide donneur comprend en outre une séquence de nucléotides intéressante qui est soit en amont (5') et liée de manière fonctionnelle au bras d'homologie, soit en aval (3') et liée de manière fonctionnelle au bras d'homologie.

4. Procédé selon la revendication 3, dans lequel la séquence de nucléotides intéressante est insérée dans le locus génomique ciblé de la cellule fongique, optionnellement dans lequel la séquence de nucléotides intéressante insérée comprend une modification de génome choisie dans le groupe constitué de: une délétion d'ADN, une disruption d'ADN, une insertion d'ADN, une inversion d'ADN, une mutation ponctuelle d'ADN, un remplacement d'ADN, une activation d'ADN, une inactivation d'ADN et un affaiblissement d'ADN.

5. Procédé selon la revendication 1, dans lequel le bras d'homologie est d'entre 100 et 40 nucléotides de longueur.

6. Procédé selon la revendication 1, dans lequel le polynucléotide donneur comprend un bras d'homologie en amont (5') et lié de manière fonctionnelle à une séquence de nucléotides intéressante et un bras d'homologie en aval (3') et lié de manière fonctionnelle à la même séquence de nucléotides intéressante, dans lequel au moins un des deux bras d'homologie est de moins de 150 nucléotides de longueur.

7. Procédé selon la revendication 6, dans lequel la séquence de nucléotides intéressante est insérée dans le locus génomique ciblé de la cellule fongique.

8. Procédé selon la revendication 6, dans lequel au moins un bras d'homologie est d'entre 100 et 40 nucléotides de longueur.

9. Procédé selon la revendication 6, dans lequel les deux bras d'homologie sont de moins de 500 nucléotides.

10. Procédé selon la revendication 3 ou la revendication 6, dans lequel la séquence de nucléotides intéressante comprend au moins un nucléotide hétérologue ou dans lequel la séquence de nucléotides intéressante comprend une séquence de polynucléotide hétérologue.

11. Procédé selon la revendication 1, dans lequel la Cas endonucléase est une Cas nickase ou un variant fonctionnel de celle-ci.

12. Procédé selon la revendication 1, dans lequel la Cas endonucléase est une Cas9 endonucléase ou un variant fonctionnel de celle-ci, optionnellement dans lequel la Cas9 endonucléase est une espèce choisie dans le groupe constitué de: *Streptococcus sp., Campylobacter sp., Neisseria sp., Francisella sp.* et *Pasteurella sp.*

13. Procédé selon la revendication 1, dans lequel:
(i) l'étape d'introduction comprend l'introduction d'une construction de polynucléotide comprenant une cassette d'expression pour exprimer la Cas endonucléase ou un variant fonctionnel de celle-ci dans la cellule fongique;
(ii) l'étape d'introduction comprend l'introduction d'une construction de polynucléotide comprenant une cassette d'expression pour exprimer l'ARN de guidage dans la cellule fongique;
(iii) l'étape d'introduction comprend l'introduction dans la cellule fongique d'une construction de polynucléotide circulaire comprenant une cassette d'expression pour la Cas endonucléase, une cassette d'expression pour l'ARN de guidage, et l'ADN donneur; ou
(iv) l'étape d'introduction comprend directement l'introduction du polynucléotide de guidage ou de la Cas endonucléase dans la cellule fongique.

14. Procédé selon la revendication 1, dans lequel la Cas endonucléase ou un variant fonctionnel de celle-ci est lié de manière fonctionnelle à un signal de localisation nucléaire.

15. Procédé selon la revendication 1, dans lequel la cellule de champignon filamenteux est choisie parmi le genre constitué de: *Trichoderma, Penicillium, Aspergillus, Humicola, Chrysosporium, Fusarium, Myceliophthora, Neurospora* et *Emericella.*
